(19) Europäisches Patentamt / European Patent Office / Office européen des brevets

(11) **EP 4 227 852 B1**

(12) **EUROPEAN PATENT SPECIFICATION**

(45) Date of publication and mention
of the grant of the patent:
**13.08.2025  Bulletin 2025/33**

(21) Application number: **21878942.8**

(22) Date of filing: **19.04.2021**

(51) International Patent Classification (IPC):
**G06N 3/04** *(2023.01)*      **A61B 5/00** *(2006.01)*
**A61B 5/0295** *(2006.01)*

(52) Cooperative Patent Classification (CPC):
**G06N 3/045; A61B 5/0295; A61B 5/7264;**
**G06F 18/213; G06N 3/08;** G06F 2218/18;
Y02A 90/10

(86) International application number:
**PCT/CN2021/088021**

(87) International publication number:
**WO 2022/077888 (21.04.2022 Gazette 2022/16)**

(54) **SCATTER DIAGRAM CLASSIFICATION METHOD AND APPARATUS FOR PHOTOPLETHYSMOGRAPHY SIGNAL**

VERFAHREN UND VORRICHTUNG ZUR KLASSIFIZIERUNG VON STREUDIAGRAMMEN FÜR PHOTOPLETHYSMOGRAFIESIGNALE

PROCÉDÉ ET APPAREIL DE CLASSIFICATION DE DIAGRAMME DE DIFFUSION POUR SIGNAL DE PHOTOPLÉTHYSMOGRAPHIE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **12.10.2020   CN 202011086771**

(43) Date of publication of application:
**16.08.2023   Bulletin 2023/33**

(73) Proprietor: **Lepu Medical Technology (Beijing) Co., Ltd.**
**Beijing 102200 (CN)**

(72) Inventors:
• **CAO, Jun**
**Beijing 102200 (CN)**
• **SUN, Hongdai**
**Beijing 102200 (CN)**
• **LI, Zhe**
**Beijing 102200 (CN)**

(74) Representative: **Glück Kritzenberger Patentanwälte PartGmbB**
**Franz-Mayer-Str. 16a**
**93053 Regensburg (DE)**

(56) References cited:
WO-A1-2019/191487      WO-A1-2020/125078
CN-A- 108 784 680       CN-A- 111 248 880
CN-A- 111 291 727       CN-A- 112 070 067

## Description

This application claims the priority of the China patent application No.202011086771.9 filed in the National Intellectual Property Administration, PRC on Oct. 12, 2020, and the invention title of "Scatter Diagram Classification Method and Device for Photoplethysmography (PPG) Signal".

## FEILD OF THE INVENTION

The invention relates to the technical field of signal processing, in particular to a scatter diagram classification method and device for photoplethysmography (PPG) signal.

## BACKGROUND OF THE INVENTION

A time interval between peak points of adjacent ECG signal waveforms is regarded as a cardiac cycle length, which is called an inter-beat interval. An inter-beat interval scatter diagram (simply referred to as scatter diagram) is a two-dimensional coordinate way to identify corresponding heart rhythm characteristics by observing the data change of the inter-beat interval. The abscissa of each scatter point in the scatter diagram represents a previous inter-beat interval of a certain heart beat, and the ordinate represents a next inter-beat interval of a certain beat. By identifying the distribution regulation of scatter points in the scatter diagram, the overall dominant rhythm of the heart can be known. An RR interval scatter diagram can be used to evaluate heart rate fluctuation, autonomic nervous regulation and heart rate variability, and can also be used to diagnose arrhythmia and evaluate the prognosis of diseases. When analyzing heart rhythm characteristics by using an inter-beat interval scatter diagram, the larger the number of scatter points, the more obvious a formed typical image, and the more accurate an analysis result, which means that a long time of continuous acquisition (for example, at least half an hour) is required. However, a conventional ECG signal acquisition method is not suitable for long-time monitoring of a test object at any time.

Photoplethysmography (PPG) is a non-invasive method to detect the change of blood volume in viable tissue by photoelectric means. Cardiac impulses make the blood flow per unit area in the blood vessel change periodically, causing blood volume to change accordingly, so that a PPG signal reflecting the amount of light absorbed by blood will also change periodically, and the periodic change of the PPG signal is closely related to cardiac impulses and the blood pressure change. For PPG signals, a time interval between the maximum peak points of adjacent signal waveforms is also equal to an inter-beat interval.

WO 2019/191487 A1 discloses a scatter diagram classification method for a photoplethysmography (PPG) signal comprising: acquiring the PPG signal; performing signal sampling processing on the PPG signal according to a preset sampling frequency to generate a PPG sampling signal; and performing scatter point two-dimensional coordinate preparation processing according to the PPG sampling signal to generate a scatter point two-dimensional coordinate sequence. The PPG signal data are communicated by a PPG sensor of a wearable device worn by a user. A plurality of heartbeats from at least a portion of the PPG signal data is determined followed by determination of the heart rhythm type based on at least the plurality of heartbeats.

A heart rhythm monitoring method and device is disclosed in WO 2020/125078 A1. Further, CN 112 070 076 A discloses a text paragraph structure restoration method, device and equipment and a computer storage medium. The method comprises the steps: carrying out the recognition of a target image, and determining all textboxes in the target image and the textbox positions of all textboxes based on a recognition result of recognition; sorting the textboxes according to the textbox positions, and inputting text features of the textboxes into a preset deep learning model for training based on a sorting result of sorting; and combining the textboxes based on the training result of the training to obtain all text paragraphs corresponding to the target picture.

CN 111 291 727 A relates to a method and device for detecting signal quality by photoplethysmography. The method comprises the steps: obtaining a PPG signal, carrying out the sampling on the PPG signal according to a data sampling frequency threshold value to obtain a PPG one-dimensional data sequence, performing fragment division on the PPG one-dimensional data sequence according to a CNN input length threshold value to generate a plurality of PPG one-dimensional sub-fragments, generating a PPG data four-dimensional tensor according to the total number of segments and the PPG one-dimensional data sequence, performing PPG feature extraction by using a CNN model to generate a PPG feature four-dimensional tensor, constructing a PPG feature two-dimensional matrix, performing PPG feature binary classification probability regression calculation by using an ANN model to generate a PPG probability two-dimensional matrix [X, 2], and performing PPG signal quality judgment on the PPG signal according to the PPG probability two-dimensional matrix [X, 2] to generate a PPG signal quality detection result.

CN 108 784 680 A describes convolutional neural networks for heart rate signal analysis including classifying scatter diagrams of ECG signals using such convolutional neural networks.

Finally, CN 111 248 880 A discloses a blood pressure prediction method based on a photoplethysmography (PPG) signal and a device. The method includes the steps: generating a PPG signal segment; performing signal decomposition on the PPG signal segment by using a continuous wavelet transform manner according an acquired wavelet base type, contraction-expansion factor and movement factor to generate a PPG wavelet coefficient matrix; performing modulo operation on matrix elements to convert the PPG wavelet coefficient matrix, and performing normalization processing on the converted matrix to generate a PPG normalized matrix; obtaining an RGB color disk matrix, and performing tensor conversion on the PPG normalized matrix according to the RGB color disk matrix to generate a PPG time-frequency three-dimensional tensor; performing tensor shape reconstruction on the PPG time-frequency three-dimensional tensor by using a bicubic interpolation algorithm according to a convolutionalnetwork input width threshold value to generate a PPG convolution three-dimensional tensor; and performing classification regression calculation on the PPG convolutional three-dimensional tensor by using a convolutional neural network classification regression model to generate PPG predicted blood pressure data pairs.

## SUMMARY OF THE INVENTION

The purpose of the present invention is to provide a scatter diagram classification method and device for a photoplethysmography (PPG) signal, electronic equipment, a computer program product and a computer readable storage medium to overcome the defects of the prior art. By extracting inter-beat interval data from PPG signals to generate a scatter diagram, and then introducing the scatter diagram into an artificial intelligence network for confirming the type of the scatter diagram for type confirmation, so as to reduce the scatter diagram generation difficulty, and enrich the application scenarios of PPG in the field of health monitoring.

In order to achieve the above object, the first aspect of the embodiment of the invention provides a computer-implemented scatter diagram classification method for a PPG signal, comprising:

acquiring the PPG signal;
performing signal sampling processing on the PPG signal according to a preset sampling frequency to generate a PPG sampling signal;
performing scatter point two-dimensional coordinate preparation processing according to the PPG sampling signal to generate a scatter point two-dimensional coordinate sequence;
performing scatter diagram resolution confirmation processing according to the sampling frequency and a preset maximum value of an inter-beat interval to generate a scatter diagram resolution;
performing scatter diagram initialization processing according to the scatter diagram resolution to generate a scatter diagram two-dimensional tensor;
performing scatter point marking processing on the scatter diagram two-dimensional tensor according to the scatter point two-dimensional coordinate sequence;
using a convolutional neural network of an artificial intelligence network to perform multilayer convolution pooling calculation on the scatter diagram two-dimensional tensor to generate a four-dimensional output tensor, the artificial intelligence network comprising the convolutional neural network, a fully connected neural network and a normalization processing layer;
using the fully connected neural network of the artificial intelligence network to perform multilayer full connection calculation on the four-dimensional output tensor to generate a two-dimensional output tensor;
using the normalization processing layer of the artificial intelligence network to perform normalization index calculation on the two-dimensional output tensor to generate a normalization two-dimensional tensor; and
performing classification confirmation processing according to the normalization two-dimensional tensor to generate confirmation data, the confirmation data comprising classification reasonable information and classification unreasonable information, wherein

performing scatter point two-dimensional coordinate preparation processing according to the PPG sampling signal to generate a scatter point two-dimensional coordinate sequence specifically comprises: sequentially extracting time information corresponding to maximum amplitude signal points of signal waveforms from the PPG sampling signal to generate peak time data, and forming a peak time data sequence from the peak time data, the PPG sampling signal comprising a plurality of the signal waveforms, the signal waveform comprising a plurality of signal points, and the number of the peak time data of the peak time data sequence is a first total number n; taking the peak time data corresponding to the first index i' as current a peak time data in the peak time data sequence, performing absolute difference calculation on the current peak time data and peak time data before the current peak time data to generate a scatter point abscissa $X_i$ corresponding to the second index i, and performing absolute difference calculation on the current peak time data and peak time data after the current peak time data to generate a scatter point ordinate $Y_i$ corresponding to the second index i, scatter point two-dimensional coordinates $XY_i$ being composed of the scatter point abscissa $X_i$ and the scatter point ordinate $Y_i$, the scatter point two-dimensional coordinates $XY_i$ being $(X_i, Y_i)$, the first index i' being an index number of the peak time data, and the value range of the first index i' is 2 to n-1, the second index i being an index number of the scatter point two-dimensional coordinates $XY_i$, i=i'-1, and the value range of the second index i is 1 to n-2; n-2 scatter point two-dimensional coordinates $XY_i$ forming the scatter point two-dimensional coordinate sequence, wherein the scatter point two-dimensional coordinate sequence being $(XY_1,...XY_i,...X-$

$Y_{n-2}$); and

only keeping one of the plurality of identical scatter point two-dimensional coordinates $XY_i$ in the scatter point two-dimensional coordinate sequence, deleting the scatter point two-dimensional coordinate $XY_i$ of which the scatter point abscissa $X_i$ exceeds the maximum value of inter-beat interval, and deleting the scatter point two-dimensional coordinate $XY_i$ of which the scatter point ordinate $Y_i$ exceeds the maximum value of the inter-beat interval.

Preferably, performing scatter diagram resolution confirmation processing according to the sampling frequency and a preset maximum value of inter-beat interval to generate a scatter diagram resolution specifically comprises:

performing maximum pixel number calculation according to the sampling frequency and the maximum value of the inter-beat interval to generate a maximum number of pixels a, a=sampling frequency * maximum value of inter-beat interval; and
the number of horizontal pixels X of the scatter diagram resolution is set to the maximum number of pixels a, the number of vertical pixels Y of the scatter diagram resolution is set to the maximum number of pixels a, and the scatter diagram resolution = X*Y = a*a.

Preferably, performing scatter diagram initialization processing according to the scatter diagram resolution to generate a scatter diagram two-dimensional tensor specifically comprises:
setting the scatter diagram two-dimensional tensor according to the scatter diagram resolution, the shape of the scatter diagram two-dimensional tensor being $H_1 \times W_1$, $H_1$ being a two-dimensional parameter of the scatter diagram two-dimensional tensor, and $H_1 = Y = a$, the $W_1$ being a one-dimensional parameter of the scatter diagram two-dimensional tensor, and $W_1 = X = a$, the scatter diagram two-dimensional tensor comprising $H_1 * W_1$ pixel data $D_{S,Z}$, and the value of the pixel data $D_{S,Z}$ being a preset first pixel value, the S being a horizontal subscript of the pixel data $D_{S,Z}$, the value range of S is 1 to $W_1$, Z being a vertical subscript of the pixel data $D_{S,Z}$, and the value range of Z is 1 to $H_1$.

Preferably, performing scatter point marking processing on the scatter diagram two-dimensional tensor according to the scatter point two-dimensional coordinate sequence specifically comprises:
in the scatter diagram two-dimensional tensor, the value of the pixel data $D_{S,Z}$ matched with the scatter point two-dimensional coordinates $XY_i$ of the scatter point two-dimensional coordinate sequence is set to the preset second pixel value, the horizontal subscript S of the pixel data $D_{S,Z}$ being the same as the scatter point abscissa $X_i$ of the scatter point two-dimensional coordinates $XY_i$, the vertical subscript Z of the pixel data $D_{S,Z}$ being the same as the scatter point ordinate $Y_i$ of the scatter point two-dimensional coordinates $XY_i$.

Preferably, using a convolutional neural network of an artificial intelligence network to perform multi-layer convolution pooling calculation on the scatter diagram two-dimensional tensor to generate a four-dimensional output tensor specifically comprises:

according to a four-dimensional tensor input data format of the convolutional neural network, raising the shape of the scatter diagram two-dimensional tensor from a two-dimensional tensor shape to a four-dimensional tensor shape to generate a four-dimensional input tensor, the shape of the four-dimensional input tensor being $B_1 \times H_2 \times W_2 \times C_1$, the $B_1$ being a four-dimensional parameter of the four-dimensional input tensor, and $B_1 = 1$, the $H_2$ being a three-dimensional parameter of the four-dimensional input tensor, and $H_2 = H_1$, the $W_2$ being a two-dimensional parameter of the four-dimensional input tensor, and $W_2 = W_1$, the $C_1$ being a one-dimensional parameter of the four-dimensional input tensor, and $C_1 = 1$; and
sending the four-dimensional input tensor into a first convolutional network layer of the convolutional neural network for first-layer convolution pooling calculation to generate a first four-dimensional tensor, then sending the first four-dimensional tensor into a second convolutional network layer of the convolutional neural network for second-layer convolution pooling calculation to generate a second four-dimensional tensor, until finally, sending a penultimate four-dimensional tensor into a last convolutional network layer of the convolutional neural network for last-layer convolution pooling calculation to generate the four-dimensional output tensor, the convolutional neural network comprising a plurality of convolutional network layers, the convolutional network layer comprising a convolution layer and a pooling layer, the shape of the four-dimensional output tensor being $B_2 \times H_3 \times W_3 \times C_2$, the $B_2$ being a four-dimensional parameter of the four-dimensional output tensor, and $B_2 = B_1 = 1$, the $H_3$ being a three-dimensional parameter of the four-dimensional output tensor, the $W_3$ being a two-dimensional parameter of the four-dimensional output tensor, and the $C_2$ being a one-dimensional parameter of the four-dimensional output tensor,
wherein sending the four-dimensional input tensor into a first convolutional network layer of the convolutional neural network for first-layer convolution pooling calculation to generate a first four-dimensional tensor is to send the four-dimensional input tensor to a first convolution layer of the first convolutional network layer for first convolution calculation to generate a first convolution four-dimensional tensor, and then send the first convolution four-dimensional tensor into a first pooling layer of the first convolutional network layer for first pooling calculation to generate the first four-dimensional tensor.

Preferably, using the fully connected neural network of the artificial intelligence network to perform multilayer full connection calculation on the four-dimensional output tensor to generate a two-dimensional output tensor specifically comprises:

according to a two-dimensional tensor input data format of the fully connected neural network, reducing the shape of the four-dimensional output tensor from a four-dimensional tensor shape to a two-dimensional tensor shape to generate a two-dimensional input tensor, the shape of the two-dimensional input tensor being $B_3 \times W_4$, the $B_3$ being a two-dimensional parameter of the two-dimensional input tensor, and $B_3=B_2=1$, the $W_4$ being a one-dimensional parameter of the two-dimensional input tensor, and $W_4= H_3*W_3*C_2$; and
sending the two-dimensional input tensor into a first fully connected layer of the fully connected neural network for first-layer full connection calculation to generate a first two-dimensional tensor, then sending the first two-dimensional tensor to a second fully connected layer of the fully connected neural network for second-layer full connection calculation to generate a second two-dimensional tensor, until finally sending a penultimate two-dimensional tensor into a last fully connected layer of the fully connected neural network for last-layer full connection calculation to generate the two-dimensional output tensor, the fully connected neural network comprising a plurality of fully connected layers, the shape of the two-dimensional output tensor being $B_4 \times W_5$, the $B_4$ being a two-dimensional parameter of the two-dimensional output tensor, and $B_4=B_3=1$, the $W_5$ being a one-dimensional parameter of the two-dimensional output tensor, and $W_5=2$, and the two-dimensional output tensor comprising two data: classification reasonable weight data and classification unreasonable weight data.

Preferably, using the normalization processing layer of the artificial intelligence network to perform normalization index calculation on the two-dimensional output tensor to generate a normalization two-dimensional tensor specifically comprises:

sending the two-dimensional output tensor into the normalization processing layer for normalization index calculation of the classification reasonable weight data and the classification unreasonable weight data to generate a classification reasonable probability and a classification unreasonable probability, the sum of the classification reasonable probability and the classification unreasonable probability being 1; and

forming the normalization two-dimensional tensor by the classification reasonable probability and the classification unreasonable probability, the shape of the normalization two-dimensional tensor being $B_5 \times W_6$, the $B_5$ being a two-dimensional parameter of the normalization two-dimensional tensor, and $B_5=B_4=1$, the $W_6$ being a one-dimensional parameter of the normalization two-dimensional tensor, and $W_6=W_5=2$, and the normalization two-dimensional tensor comprising two data: the classification reasonable probability and the classification unreasonable probability.

Preferably, performing classification confirmation processing according to the normalization two-dimensional tensor to generate confirmation data (the confirmation data comprising classification reasonable information and classification unreasonable information) specifically comprises:

taking the classification reasonable information as the confirmation data when the classification reasonable probability is higher than the classification unreasonable probability; and
taking the classification unreasonable information as the confirmation data when the classification reasonable probability is lower than the classification unreasonable probability.

A second aspect of the embodiment of the invention provides a scatter diagram classification device for a PPG signal, comprising:

an acquisition module for acquiring the PPG signal;
a sampling module for performing signal sampling processing on the PPG signal according to a preset sampling frequency to generate a PPG sampling signal;
a scatter diagram module for performing scatter point two-dimensional coordinate preparation processing according to the PPG sampling signal to generate a scatter point two-dimensional coordinate sequence; performing scatter diagram resolution confirmation processing according to the sampling frequency and a preset maximum value of an interbeat interval to generate a scatter diagram resolution; performing scatter diagram initialization processing according to the scatter diagram resolution to generate a scatter diagram two-dimensional tensor; and performing scatter point marking on the scatter diagram two-dimensional tensor according to the scatter point two-dimensional coordinate sequence;
an artificial intelligence calculation module for using a convolutional neural network of an artificial intelligence network to perform multilayer convolution pooling calculation on the scatter diagram two-dimensional tensor to generate a four-dimensional output tensor; using a fully connected neural network of the artificial intelligence network to perform multi-layer full connection calculation on the four-dimensional output tensor to generate a two-dimensional output tensor; and using a normalization processing layer of the artificial intelligence network to perform normalization index calculation on the two-dimensional output tensor to generate a normalization two-dimensional tensor, the artificial intelligence network comprising the convolutional neural network, the fully connected neural network and the normalization processing layer; and

a classification confirmation module for performing classification confirmation processing according to the normalization two-dimensional tensor to generate confirmation data, the confirmation data comprising classification reasonable information and classification unreasonable information.

A third aspect of the embodiment of the invention provides a computer program product, which comprises a computer program code that, when executed by a computer, causes the computer to perform the method described in the first aspect.

A fourth aspect of the embodiment of the invention provides a computer-readable storage medium, which stores computer instructions that, when executed by a computer, cause the computer to execute the method described in the first aspect.

The embodiment of the invention provides a scatter diagram classification method and device for a PPG signal, a computer program product and a computer readable storage medium. By extracting inter-beat interval data from PPG signals to generate a scatter diagram, and then introducing the scatter diagram into an artificial intelligence network for confirming the type of the scatter diagram for type confirmation, therefore the scatter diagram generation difficulty is reduced, and the application scenarios of PPG in the field of health monitoring are enriched.

**BRIEF DESCRIPTION OF THE DRAWINGS**

Fig. 1 is a diagram of a scatter diagram classification method for a photoplethysmography (PPG) signal provided in the first embodiment of the invention,
Fig. 2 is a diagram of a PPG sampling signal provided in the first embodiment of the invention,
Fig. 3 is a scatter diagram provided by the first embodiment of the invention,
Fig. 4a is a structural schematic diagram of an artificial intelligence network provided in the first embodiment of the invention,
Fig. 4b is a structural schematic diagram of a convolutional neural network provided in the first embodiment of the invention,
Fig. 4c is a structural schematic diagram of a fully connected neural network provided by the first embodiment of the invention,
Fig. 5 is a modular structure diagram of a scatter diagram classification device for a photoplethysmography (PPG) signal provided in the second embodiment of the invention; and
Fig. 6 is a structural schematic diagram of electronic equipment provided by the third embodiment of the invention.

**DETAILED DESCRIPTION OF THE INVENTION**

In order to make the object, technical solution and advantages of the invention clearer, the invention will be described in further detail below with reference to the accompanying drawings. The invention is defined by the claims.

Before the embodiment of the invention is described in detail, the structure and data formats of an artificial intelligence network for confirming scatter diagram classification mentioned above are briefly described.

After the artificial intelligence network performs corresponding classification confirmation calculation on a scatter diagram, two weight data are obtained: the classification reasonable weight data and the classification unreasonable weight data. The embodiment of the present invention finally obtains a classification confirmation result (reasonable classification and unreasonable classification) from the two weight data. For example, when the artificial intelligence network is specifically an atrial fibrillation scatter diagram confirmation network, atrial fibrillation scatter diagram classification confirmation calculation is performed for the current scatter diagram. When the classification confirmation result is reasonable classification, it is considered that the current scatter diagram is an atrial fibrillation scatter diagram, and when the classification confirmation result is unreasonable classification, it is considered that the current scatter diagram is not an atrial fibrillation scatter diagram.

The artificial intelligence network here is composed of a convolutional neural network, a fully connected neural network and a normalization processing layer. The convolutional neural network is composed of a plurality of convolutional network layers, and the fully connected neural network is composed of a plurality of fully connected layers.

The convolutional neural network is used to perform convolution calculation and pooling calculation of the scatter diagram, and outputs feature data for further learning and calculation by other networks. The convolutional neural network is composed of a plurality of convolutional network layers, and the output data of each convolutional network layer will be used as the input data of the next convolutional network layer. Each convolutional network layer consists of one convolution layer and one pooling layer. Wherein, the function of the convolution layer is convolution operation, and the purpose of convolution operation is to extract the features of the input data. The convolution layer of the first convolutional network layer extracts some low-level features (such as edges, lines, and angles), and the convolution layers of subsequent convolutional network layers will continuously iterate from low-level features and extract more complex high-level features. The pooling layer has two functions: one is to keep translation, rotation and scale invariance, and there are two commonly used processing methods: mean-pooling and max-pooling; the other function is to reduce the number of parameters and calculation amount of the next convolution layer on the premise of keeping the main features of the output data of the previous convolution layer, so as to prevent over-fitting.

Here, in each convolutional network layer, the scatter diagram will perform convolution operation with a convolution kernel with a fixed size. Generally, the shape of the convolution kernel is $3\times3$, $5\times5$, and $7\times7$. After each convolutional network layer, the output data will have a shape change relative to the input data, but still keep the four-dimensional tensor form: the input four-dimensional tensor (for example, the shape is $P_{in4}\times P_{in3}\times P_{in2}\times P_{in1}$), and the output four-dimensional tensor (for example, the shape is $P_{out4}\times P_{out3}\times P_{out2}\times P_{out1}$), wherein the $P_{in4}$, $P_{in3}$, $P_{in2}$ and $P_{in1}$ are four-dimensional, three-dimensional, two-dimensional and one-dimensional parameters of the input four-dimensional tensor respectively, and the $P_{out4}$, $P_{out3}$, $P_{out2}$ and $P_{out1}$ are four-dimensional, three-dimensional, two-dimensional and one-dimensional parameters of the output four-dimensional tensor respectively. After the calculation of each convolutional network layer, the shape of the output four-dimensional tensor is subjected to the following change in terms of dimensional parameters relative to the shape of the input four-dimensional tensor: (1) $P_{out4}$ vs $P_{in4}$, the four-dimensional parameter does not change and is always 1 in the embodiment of the present invention; (2) $P_{out3}$ and $P_{out2}$ vs $P_{in3}$ and $P_{in2}$, the three-dimensional parameter and the two-dimensional parameter change, which is related to the size of the convolution kernel and the setting of a sliding step of each convolution layer, as well as the size of a pooling window and a sliding step of the pooling layer; and (3) $P_{out1}$ vs $P_{in1}$, the one-dimensional parameter changes, which is related to a selected output space dimension (the number of convolution kernels) in the convolution layer.

An output result of the convolutional neural network will be input into the fully connected layer of the fully connected neural network for the full connection calculation. The fully connected layer is a complex network structure formed by a large number of neurons (nodes) connected to each other, and is some kind of abstraction, simplification and simulation of the organization structure and operation mechanism of the human brain. Each fully connected layer comprises a plurality of nodes, each node is connected with all nodes of the previous layer to synthesize the node data extracted from the previous layer for one node calculation, and the calculation result is taken as the value of the current node to be connected to and acquired by nodes of the next fully connected layer. The node calculation here is also called the full connection calculation. Here, the input data and output data formats of each fully connected layer are two-dimensional tensors: the input two-dimensional tensor (for example, in the shape of $Q_{in2}\times Q_{in1}$) and the output two-dimensional tensor (in the shape of $Q_{out2}xQ_{out1}$), wherein $Q_{in2}$ and $Q_{in1}$ are two-dimensional and one-dimensional parameters of the input two-dimensional tensor, and $Q_{out2}$ and $Q_{out1}$ are two-dimensional and one-dimensional parameters of the output two-dimensional tensor. After the calculation of each fully connected layer, the shape of the output two-dimensional tensor is subjected to the following change in terms of dimensional parameters relative to the shape of the input two-dimensional tensor: (1) $Q_{out2}$ vs $Q_{in2}$, the two-dimensional parameter does not change and is always 1 in the embodiment of the present invention; and (2) $Q_{out1}$ vs $Q_{in1}$, the change of the one-dimensional parameter is related to the total number of nodes in the fully connected layer. Specifically, the total number of nodes of the last fully connected layer in the embodiment of the present invention is 2, and $Q_{out1}$ is 2 accordingly.

It can be seen from the above that the output of the convolutional neural network is a four-dimensional tensor, and the input of the fully connected neural network is a two-dimensional tensor, so it is necessary to perform the reduce dimension processing on the output result of the convolutional neural network layer from the output four-dimensional tensor shape ($P_{out4}\times P_{out3}\times P_{out2}\times P_{out1}$) to the input two-dimensional tensor shape ($Q_{in2}xQ_{in1}$) of the fully connected neural network. The two-dimensional tensor shape finally output by the fully connected neural network should be $1\times2$, and the two data in the two-dimensional tensor are the two weight data obtained after the artificial intelligence network performs classification confirmation calculation on the scatter diagram: the classification reasonable weight data and the classification unreasonable weight data.

After the fully connected neural network outputs the two weight data, the normalization processing layer is used to normalize the weight data. Specifically, the normalization processing layer will use a Softmax function (the normalization exponential function) to perform normalization exponential calculation on the two weight data, and convert the two weight data into two probabilities less than 1, so as to facilitate subsequent classification confirmation judgment. Here, the two probabilities are: classification reasonable probability and classification unreasonable probability, and the sum of the two probability data should be 1.

Finally, the embodiment of the present invention will select the one with a larger value from the two probabilities as a classification confirmation result, that is, if the one with a larger value is the classification reasonable probability, then the classification result is reasonable classification, and if the one with a larger value is the classification unreasonable probability, then the classification result is unreasonable classification.

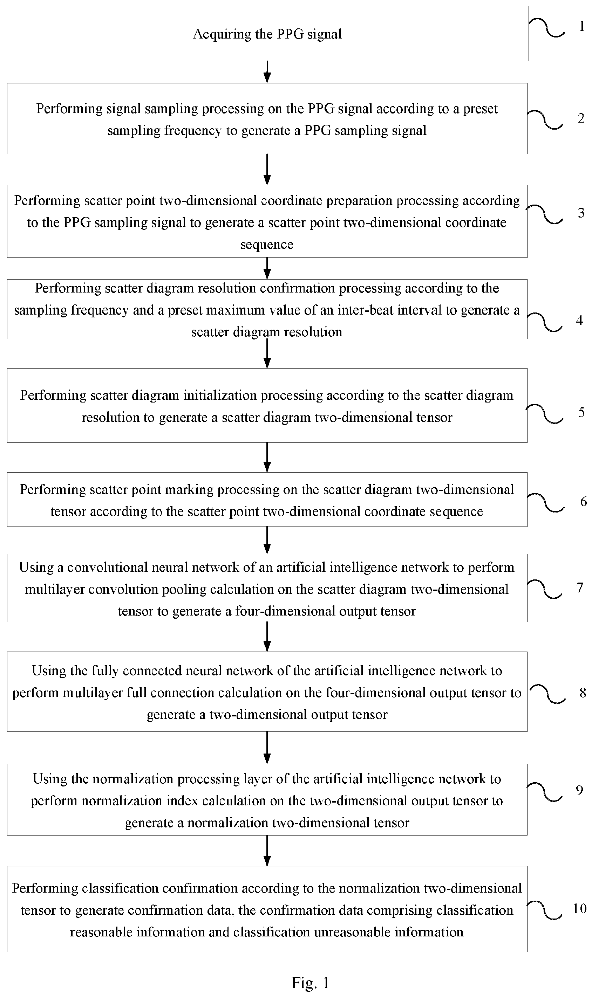

According to the scatter diagram classification method for a PPG signal provided by the first embodiment of the present invention, inter-beat interval data are extracted from PPG signals to generate a scatter diagram, then the scatter diagram is introduced into an artificial intelligence network for confirming the type of the scatter diagram for type confirmation, and finally the classification confirmation result is obtained according to the weight data output by the artificial intelligence network. As shown in Fig. 1 which is a diagram of a scatter diagram classification method for a PPG signal provided in the first embodiment of the invention, the method mainly comprises the following steps.

Step 1, acquiring the PPG signal.

Specifically, the equipment may acquire a real-time PPG signal of a test object through a local PPG signal acquisition device, and may also acquire the real-time PPG signal of the test object through a PPG signal acquisition device of other equipment connected to the equipment itself. And may also acquire the historical PPG signal of the test object from a local storage medium or a storage medium of other equipment connected to the equipment itself.

Here, the equipment is specifically a terminal equipment or a server for implementing the method provided by the embodiment of the present invention.

For example, the acquired PPG signal is a real-time PPG signal with a length of 30 minutes.

Step 2, performing signal sampling processing on the PPG signal according to a preset sampling frequency to generate a PPG sampling signal.

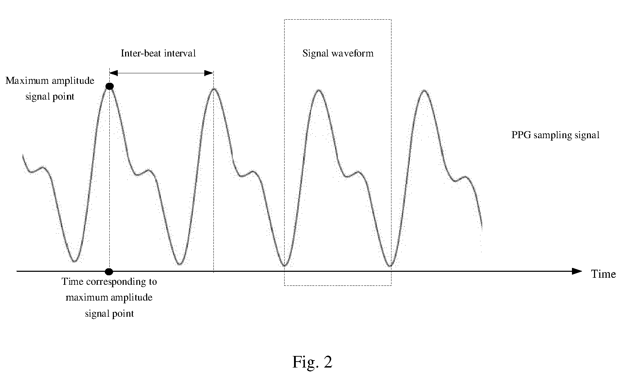

Here, the sampling frequency is used to sample the PPG signal, and the sampling frequency is stored in a local storage medium of the equipment. The PPG sampling signal, as shown in the diagram of a PPG sampling signal provided in the first embodiment of the invention in Fig. 2, comprises a plurality of signal waveforms, and each signal waveform comprises a plurality of signal points.

For example, if the length of the PPG signal is 30 minutes and the sampling frequency is 250 Hz, the length of the PPG sampling signal is also 30 minutes, and there are 250 * 60 * 30 = 450,000 signal points.

Step 3, performing scatter point two-dimensional coordinate preparation processing according to the PPG sampling signal to generate a scatter point two-dimensional coordinate sequence.

Specifically including the following steps: step 31, sequentially extracting time information corresponding to maximum amplitude signal points of signal waveforms from the PPG sampling signal to generate peak time data, and forming a peak time data sequence from the peak time data,

wherein the number of the peak time data of the peak time data sequence is a first total number n.

Here, as shown in Fig. 2, in each signal waveform, a signal point with the largest signal amplitude is the maximum amplitude signal point, and the time information corresponding to the maximum amplitude signal point is the peak time data.

For example, if the length of the PPG sampling signal is 30 minutes and there are 1800 maximum amplitude signal points, then the first total number n=1800, and the peak time data sequence comprises 1800 peak time data.

Step 32, taking the peak time data corresponding to the first index i' as current peak time data in the peak time data sequence, performing absolute difference calculation on the current peak time data and a peak time data before the current peak time data to generate a scatter point abscissa $X_i$ corresponding to the second index i, and performing absolute difference calculation on the current peak time data and peak time data after the current peak time data to generate a scatter point ordinate $Y_i$ corresponding to the second index i; and forming scatter point two-dimensional coordinates $XY_i$ by the scatter point abscissa $X_i$ and the scatter point ordinate $Y_i$.

Wherein the scatter point two-dimensional coordinates $XY_i$ are $(X_i, Y_i)$, the first index i' is an index number of the peak time data, the value range of the first index i' is 2 to n-1, the second index i is an index number of the scatter point two-dimensional coordinates $XY_i$, i=i'-1, and the value range of the second index i is 1 to n-2.

Here, as shown in Fig. 2, the absolute difference between two adjacent peak time data is the inter-beat interval. because the scatter point two-dimensional coordinates of the scatter diagram are generated by taking a previous inter-beat interval of certain peak time data as the scatter point abscissa and a next inter-beat interval as the scatter point ordinate, when selecting the previous peak time data, it is necessary to exclude the first peak time data (without a previous peak time data) and the nth peak time data (without a next peak time data), so the value range of the first index i' is 2 to n-1. In addition, since the calculated scatter point two-dimensional coordinates need to be counted from the first coordinates, thereby the second index i=i'+1, herein the second index i is used to identify the first to the n-2th scatter point two-dimensional coordinates.

For example, if the first total number n=1800 and the peak time data sequence comprises 1800 peak time data, then,

when i'=2, the second peak time data is used as the current peak time data, the first peak time data is used as the previous peak time data, the third peak time data is used as the next peak time data, the generated the ith (here i=i'-1=2-1=1) scatter point two-dimensional coordinates $XY_1$ is $(X_1, Y_1)$, where the scatter point abscissa $X_1$=|second peak time data - first peak time data|, and the scatter point ordinate $Y_1$=|third peak time data - second peak time data|, here ‖ is an absolute value operator.

When i'=3, the third peak time data is used as the current peak time data, the second peak time data is used as the previous peak time data, the fourth peak time data is used as the next peak time data, the generated the ith (here i=i'-1=3-1=2) scatter point two-dimensional coordinates $XY_2$ is $(X_2,Y_2)$, where the scatter point abscissa $X_2$ = |third peak time data-second peak time data|, and the scatter point ordinate $Y_2$ = |fourth peak time data-third peak time data|.

And so on,

when i'=n-1=1800-1=1799, the 1799th peak time data is used as the current peak time data, the 1798th peak time data is used as the previous peak time data, the 1800th peak time data is used as the next peak time data, the generated ith (here i=i'-1=1799-1=1798) scatter point two-dimensional coordinates $XY_{1798}$ is $(X_{1798}, Y_{1798})$, where the scatter point abscissa $X_{1798}$=|1799th peak time data-1798th peak time data|, and the scatter point ordinate $Y_{1798}$=|1800th peak time data-1799th peak time data|.

Step 33, forming the scatter point two-dimensional coordinate sequence by n-2 scatter point two-dimensional coordinates $XY_i$.

Wherein the scatter point two-dimensional coordinate sequence is $(XY_1,...XY_i,...XY_{n-2})$.

Here, the total number of scatter points obtained by the PPG sampling signal is n-2.

For example, if the first total number n=1800 and the peak time data sequence comprises 1800 peak time data, then the scatter point two-dimensional coordinate sequence is $(XY_1,...XY_i,...XY_{1798})$.

Step 34, only keeping one of the plurality of identical scatter point two-dimensional coordinates $XY_i$ in the scatter point two-dimensional coordinate sequence, deleting the scatter point two-dimensional coordinate $XY_i$ of which the scatter point abscissa $X_i$ exceeds the maximum value of inter-beat interval, and deleting the scatter point two-dimensional coordinate $XY_i$ of which the scatter point ordinate $Y_i$ exceeds the maximum value of the inter-beat interval.

Here, the maximum value of the inter-beat interval is stored in the local storage medium of the equipment.

Here, clear the redundant scatter points first, which are scatter points with the same scatter point two-dimensional coordinates $XY_i$. The clearing method is to keep only one of them and delete the other redundant scatter point two-dimensional coordinates $XY_i$. Then, clear the error scatter points which are too discrete. The embodiment of the present invention uses the maximum value of the inter-beat interval to identify the error scatter points. Because both the abscissa and ordinate of the scatter point two-dimensional coordinates $XY_i$ are actually the inter-beat intervals, the scatter point two-dimensional coordinates $XY_i$ of which the abscissa and ordinate exceed the maximum value of the inter-beat interval are all recorded as the error scatter point and deleted from the scatter point two-dimensional coordinate sequence.

For example, the first total number n=7, the peak time data sequence comprises 7 peak time data, and the scatter point two-dimensional coordinate sequence is $(XY_1,XY_2,XY_3,XY_4,XY_5)$, wherein $XY_1$ is (1,1), $XY_2$ is (3,1), $XY_3$ is (2,1), $XY_4$ is (1,3), $XY_5$ is (1,1), and the maximum value of the inter-beat interval is 2 seconds. First, delete one of $XY_1$ and $XY_5$ with the same coordinates (assuming $XY_5$ is deleted), then delete the $XY_2$ of which the abscissa exceeds the maximum value of the inter-beat interval and the $XY_4$ of which the ordinate exceeds the maximum value of the inter-beat interval, and finally, after redundancy removal and error removal, the scatter point two-dimensional coordinate sequence is $(XY_1,XY_3)$, which means that the total number of scatter points at this time have been changed from the previous five to two.

Step 4, performing scatter diagram resolution confirmation processing according to the sampling frequency and a preset maximum value of an inter-beat interval to generate a scatter diagram resolution.

Specifically comprising the following steps: step 41, performing maximum pixel number calculation according to the sampling frequency and the maximum value of the inter-beat interval to generate a maximum number of pixels a, a=sampling frequency * maximum value of inter-beat interval.

Here, if the scatter diagram two-dimensional tensor to be obtained later is regarded as a pixel graph, the tensor shape of the pixel graph needs to be set by calculating a resolution, the resolution is the number of horizontal pixels × the number of vertical pixels, wherein the number of horizontal pixels should be a maximum number of pixels in a horizontal axis direction and the number of vertical pixels should be a maximum number of pixels in a vertical axis direction. It is also known that a maximum boundary value between a horizontal axis and a vertical axis is the maximum value of the inter-beat interval, then after the minimum accuracy in the horizontal axis direction is obtained, the maximum number of pixels in the horizontal axis direction=the maximum boundary value/the minimum accuracy in the horizontal axis direction, and similarly, the maximum number of pixels in the vertical axis direction=the maximum boundary value/the minimum accuracy in the vertical axis direction. Because the interval between two signal points on the PPG sampling signal should be 1/sampling frequency (second), and both the minimum value and minimum accuracy of the inter-beat interval should be 1/ sampling frequency, that is, the minimum accuracy in the horizontal axis direction and the vertical axis direction should be 1/ sampling frequency. Therefore,

the maximum number of pixels in the horizontal axis direction = the maximum boundary value/the minimum accuracy in the horizontal axis direction = maximum boundary value /(1/ sampling frequency) =maximum boundary value * sampling frequency, and

the maximum number of pixels in the horizontal axis direction=the maximum boundary value/the minimum accuracy in the vertical axis direction=maximum boundary value /(1/ sampling frequency)=maximum boundary value * sampling frequency.

Because the maximum number of pixels in the horizontal axis direction is equal to the maximum number of pixels in the vertical axis direction, the calculation of the maximum number a of pixels is only done once in step 41.

For example, if the maximum value of the inter-beat interval is 2 seconds and the sampling frequency is 250 Hz, the maximum number of pixels a=2 * 250=500.

Step 42, setting the number of horizontal pixels X of the scatter diagram resolution as the maximum number of pixels a, and setting the number of vertical pixels Y of the scatter diagram resolution as the maximum number of pixels a.

Here, the scatter diagram resolution=X * Y=a * a.

As mentioned above, the resolution is the number of horizontal pixels $\times$ the number of vertical pixels.

For example, if the maximum value of the inter-beat interval is 2 seconds, the sampling frequency is 250 Hz, and the maximum number of pixels a=500, then the scatter diagram resolution is $500 \times 500$.

Step 5, performing scatter diagram initialization processing according to the scatter diagram resolution to generate a scatter diagram two-dimensional tensor.

Specifically comprising: setting a scatter diagram two-dimensional tensor according to the scatter diagram resolution.

Wherein the shape of the scatter diagram two-dimensional tensor is $H_1 \times W_1$, $H_1$ is a two-dimensional parameter of the scatter diagram two-dimensional tensor, and $H_1=Y=a$, $W_1$ is a one-dimensional parameter of the scatter diagram two-dimensional tensor, and $W_1=X=a$, the scatter diagram two-dimensional tensor comprises $H_1*W_1$ pixel data $D_{S,Z}$, a value of the pixel data $D_{S,Z}$ is a preset first pixel value, S is a horizontal subscript of the pixel data $D_{S,Z}$, the value range of S is 1 to $W_1$, Z is a vertical subscript of the pixel data $D_{S,Z}$, and the value range of Z is 1 to $H_1$.

Here, the first pixel value is stored in the local storage medium of the equipment, and the default first pixel value is set to 0.

Here, the scatter diagram two-dimensional tensor is used to store the pixel values of all the pixels of the scatter diagram. The scatter diagram resolution is the number of horizontal pixels X $\times$ the number of vertical pixels Y($a \times a$). The shape of the scatter diagram two-dimensional tensor should correspond to the scatter diagram resolution, so the two-dimensional parameter and the one-dimensional parameter of the scatter diagram two-dimensional tensor are $H_1=Y=a$ and $W_1=X=a$ respectively. The pixel data $D_{S,Z}$ is the data in the scatter diagram two-dimensional tensor, and the specific value is a pixel value of a corresponding pixel point.

For example, if the maximum value of the inter-beat interval is 2 seconds, the sampling frequency is 250 Hz, the maximum number of pixels a=500, the scatter diagram resolution is $500 \times 500$, and the first pixel value is 0 by default, then $H_1=Y=a=500$, $W_1=X=a=500$, and the shape of the scatter diagram two-dimensional tensor is $500 \times 500$. The data of the scatter diagram two-dimensional tensor are:

$$\begin{pmatrix} D_{1,1} & \cdots & D_{500,1} \\ \cdots & D_{S,Z} & \cdots \\ D_{1,500} & \cdots & D_{500,500} \end{pmatrix}$$

Wherein the value range of the horizontal subscript S of the pixel data $D_{S,Z}$ if 1 to 500, and the value range of the vertical subscript Z of the pixel data $D_{S,Z}$ is 1 to 500. The value of each pixel data $D_{S,Z}$ is set to 0 by default. At this time, the scatter diagram two-dimensional tensor can be regarded as a blank scatter diagram.

Step 6, performing scatter point marking processing on the scatter diagram two-dimensional tensor according to the scatter point two-dimensional coordinate sequence.

Specifically comprising: in the scatter diagram two-dimensional tensor, setting the value of the pixel data $D_{S,Z}$ matched with the scatter point two-dimensional coordinates $XY_i$ of the scatter point two-dimensional coordinate sequence as a preset second pixel value.

Wherein the horizontal subscript S of the pixel data $D_{S,Z}$ is the same as the scatter point abscissa $X_i$ of the scatter point two-dimensional coordinates $XY_i$, and the vertical subscript Z of the pixel data $D_{S,Z}$ is the same as the scatter point ordinate $Y_i$ of the scatter point two-dimensional coordinates $XY_i$.

Here, the second pixel value is stored in the local storage medium of the equipment, and the default value of the second pixel value is set to 1.

Here, in the scatter diagram two-dimensional tensor output in step 6, scatter points obtained in step 3 are marked, and the basis of the marking process is the scatter point two-dimensional coordinates output in step 3.

For example, the first total number n=1800, the peak time data sequence comprises 1800 peak time data, and the scatter point two-dimensional coordinate sequence is $(XY_1,...XY_i,...XY_{1798})$, and there is no redundant or error scatter point. The maximum value of the inter-beat interval is 2 seconds, the sampling frequency is 250 Hz, the shape of the scatter diagram two-dimensional tensor is $500 \times 500$, the second pixel value is 1 by default, and the data of the scatter diagram two-dimensional tensor are:

$$\begin{pmatrix} D_{1,1} & \cdots & D_{500,1} \\ \cdots & D_{S,Z} & \cdots \\ D_{1,500} & \cdots & D_{500,500} \end{pmatrix}$$

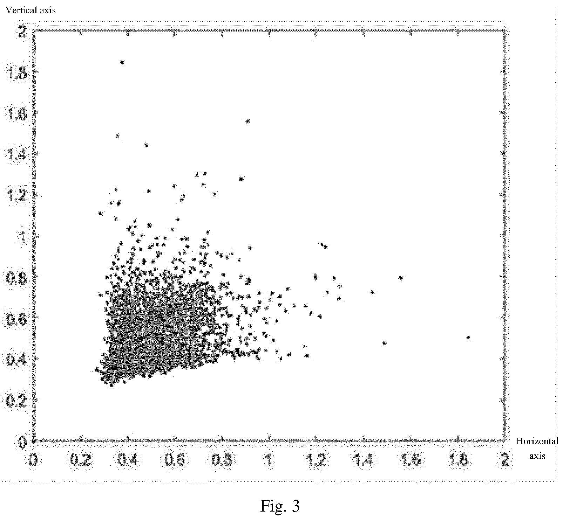

Then, in the scatter diagram two-dimensional tensor, setting the value of the pixel data $D_{S,Z}$ matched with the scatter point two-dimensional coordinates $XY_i$ of the scatter point two-dimensional coordinate sequence as a preset second pixel value is to set the value of the pixel data $D_{S,Z}$ of which data subscripts are the same as the scatter point two-dimensional coordinates as the second pixel value (set as 1) in a data area of the above two-dimensional tensor. Suppose that the scatter point two-dimensional coordinates $XY_i$ in the scatter point two-dimensional coordinate sequence is specifically (1,1), then, the value of $D_{1,1}$ is set to 1. suppose that the scatter point two-dimensional coordinates $XY_i$ in the scatter point two-dimensional coordinate sequence is specifically (9,8), then, the value of $D_{9,8}$ is set to 1, and so on, until the values of the pixel data $D_{S,Z}$ corresponding to all the scatter point two-dimensional coordinates $XY_i$ in the scatter point two-dimensional coordinate sequence is set. At this point, the scatter diagram two-dimensional tensor can be regarded as a scatter diagram that has finished scatter point marking, as shown in the scatter diagram provided by the first embodiment of the present invention in Fig. 3.

Step 7, using a convolutional neural network of an artificial intelligence network to perform multilayer convolution pooling calculation on the scatter diagram two-dimensional tensor to generate a four-dimensional output tensor.

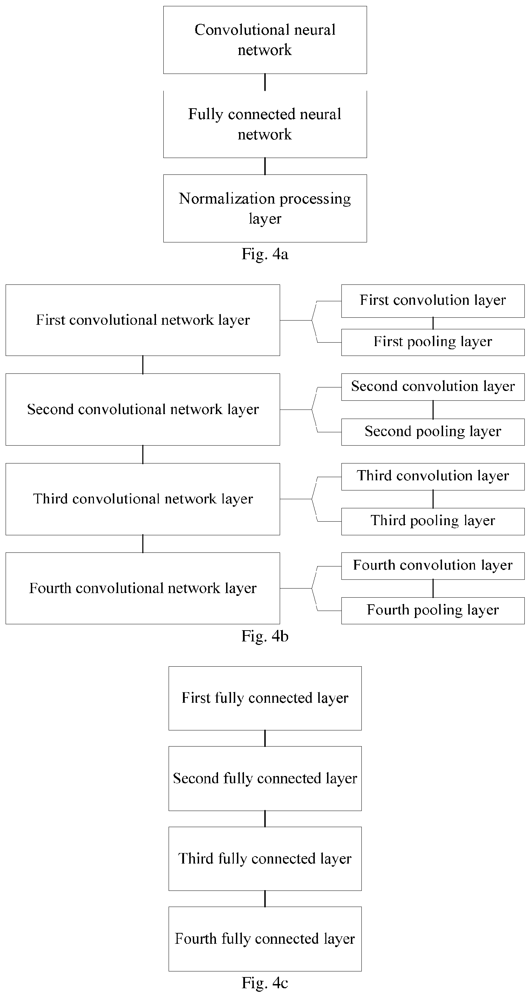

Wherein the artificial intelligence network, as shown in Fig. 4a which is a structural diagram schematic of an artificial intelligence network provided in the first embodiment of the present invention, comprises a convolutional neural network, a fully connected neural network and a normalization processing layer.

Specifically comprising: step 71, according to a four-dimensional tensor input data format of the convolutional neural network, raising the shape of the scatter diagram two-dimensional tensor from a two-dimensional tensor shape to a four-dimensional tensor shape to generate a four-dimensional input tensor.

Wherein the shape of the four-dimensional input four-dimensional tensor is $B_1 \times H_2 \times W_2 \times C_1$, $B_1$ is a four-dimensional parameter of the four-dimensional input four-dimensional tensor, and $B_1=1$, $H_2$ is a three-dimensional parameter of the four-dimensional input four-dimensional tensor, and $H_2=H_1$, $W_2$ is a two-dimensional parameter of the four-dimensional input four-dimensional tensor, and $W_2=W_1$, $C_1$ is a one-dimensional parameter of the four-dimensional input four-dimensional tensor, and $C_1=1$.

Here, the shape of the scatter diagram two-dimensional tensor is raised from the two-dimensional tensor shape to the four-dimensional tensor shape, and the process only resets the tensor shape without destroying the actual data order in the tensor.

For example, if the scatter diagram two-dimensional tensor [500,500] is raised from the two-dimensional tensor shape to the four-dimensional tensor shape, $B_1=1$, $H_2=H_1=500$, $W_2=W_1=500$, $C_1=1$, then the shape of the four-dimensional input tensor is $1 \times 500 \times 500 \times 1$, which is expressed as the four-dimensional input tensor [1,500,500,1] here.

Step 72, sending the four-dimensional input tensor into a first convolutional network layer of the convolutional neural network for first-layer convolution pooling calculation to generate a first four-dimensional tensor, then sending the first four-dimensional tensor into a second convolutional network layer of the convolutional neural network for second-layer convolution pooling calculation to generate a second four-dimensional tensor, until finally, sending a penultimate four-dimensional tensor into the last convolutional network layer of the convolutional neural network for last-layer convolution pooling calculation to generate the four-dimensional output tensor.

Wherein the convolutional neural network comprises a plurality of convolutional network layers, the convolutional network layer comprises a convolution layer and a pooling layer, the shape of the four-dimensional output tensor is $B_2 \times H_3 \times W_3 \times C_2$, $B_2$ is a four-dimensional parameter of the four-dimensional output tensor, and $B_2=B_1=1$, $H_3$ is a three-dimensional parameter of the four-dimensional output tensor, $W_3$ is a two-dimensional parameter of the four-dimensional output tensor, and $C_2$ is a one-dimensional parameter of the four-dimensional output tensor.

Wherein, the four-dimensional input tensor is sent to the first convolutional network layer of the convolutional neural network for first-layer convolution pooling calculation to generate the first four-dimensional tensor, specifically, the four-dimensional input tensor is sent to the first convolution layer of the first convolutional network layer for the first convolution calculation to generate a first convolution four-dimensional tensor, and then, the first convolution four-dimensional tensor is sent to the first pooling layer of the first convolutional network layer for the first pooling calculation to generate the first four-dimensional tensor.

For example, the convolutional neural network comprises four convolutional network layers, and its network structure is shown in a structural diagram schematic of a convolutional neural network provided by the first embodiment of the present invention in Fig. 4b, then, the four-dimensional input tensor is sent into the first convolution layer of the first convolutional network layer of the convolutional neural network for the first convolution calculation to generate a first convolution four-dimensional tensor, and the first convolution four-dimensional tensor is sent into the first pooling layer for the first pooling calculation to generate the first four-dimensional tensor.

The first four-dimensional tensor is sent into a second convolution layer of a second convolutional network layer of the convolutional neural network for the second convolution calculation to generate a second convolution four-dimensional tensor, and the second convolution four-dimensional tensor is sent into a second pooling layer for the second pooling calculation to generate a second four-dimensional tensor.

The second four-dimensional tensor is sent into a third convolution layer of a third convolutional network layer of the convolutional neural network for the third convolution calculation to generate a third convolution four-dimensional tensor, and the third convolution four-dimensional tensor is sent into a third pooling layer for the third pooling calculation to generate a third four-dimensional tensor, and

the third four-dimensional tensor is sent into a fourth convolution layer of a fourth convolutional network layer of the convolutional neural network for the fourth convolution calculation to generate a fourth convolution four-dimensional tensor, and the fourth convolution four-dimensional tensor is sent into a fourth pooling layer for the fourth pooling calculation to finally obtain the four-dimensional input tensor.

As can be seen from the foregoing, in the convolutional neural network, after each convolution layer or pooling layer, the shape of the input data will change, but the four-dimensional tensor form does not change, and the four-dimensional parameter will not change. The change of the three-dimensional and second-dimensional parameters is related to the size of a convolution kernel and the setting of a sliding step of each convolution layer, as well as the size of a pooling window and a sliding step of the pooling layer, and the change of the one-dimensional parameter is related to a selected output space dimension (the number of convolution kernels) in the convolution layer. In practical application, the setting of the number of layers in the network and various parameters of each layer may be constantly revised according to experience and experimental results.

Step 8, using the fully connected neural network of the artificial intelligence network to perform multi-layer full connection calculation on the four-dimensional output tensor to generate a two-dimensional output tensor.

Specifically comprising: step 81, according to a two-dimensional tensor input data format of the fully connected neural network, reducing the shape of the four-dimensional output tensor from a four-dimensional tensor shape to a two-dimensional tensor shape to generate a two-dimensional input tensor.

Wherein the shape of the two-dimensional input tensor is $B_3 \times W_4$, $B_3$ is a two-dimensional parameter of the two-dimensional input tensor, and $B_3=B_2=1$, $W_4$ is a one-dimensional parameter of the two-dimensional input tensor, and $W_4= H_3*W_3*C_2$.

Here, the shape of the four-dimensional output tensor is reduced from the four-dimensional tensor shape to the two-dimensional tensor shape, and the process only resets the tensor shape without destroying the actual data order in the tensor.

For example, if the shape of the output four-dimensional tensor of the convolutional neural network is $1 \times 2 \times 20 \times 64$, then $B_3=B_2=1$, $W_8=H_3*W_3*C_2=2*20*64=2560$, and the shape of the two-dimensional input tensor should be $1 \times 2560$, which is expressed here as the two-dimensional input tensor [1,2560].

Step 82, sending the two-dimensional input tensor into a first fully connected network layer of the fully connected neural network for first-layer full connection calculation to generate a first two-dimensional tensor, then sending the first two-dimensional tensor into a second fully connected network layer of the fully connected neural network for second-layer full connection calculation to generate a second two-dimensional tensor, and finally, sending a penultimate two-dimensional tensor into a last fully connected network layer of the fully connected neural network for last-layer full connection calculation to generate the two-dimensional output tensor.

Wherein the fully connected neural network comprises multiple fully connected layers, the shape of the two-dimensional output tensor is $B_4XW_5$, $B_4$ is a two-dimensional parameter of the two-dimensional output tensor, and $B_4=B_3=1$, $W_5$ is a one-dimensional parameter of the two-dimensional output tensor, and $W_5=2$, and the two-dimensional output tensor comprises two data: classification reasonable weight data and classification unreasonable weight data.

For example, the fully connected neural network comprises four fully connected layers, and its network structure is shown in Fig. 4c, which is a structural diagram schematic of a fully connected neural network provided by the first embodiment of the present invention, then

the two-dimensional input tensor is sent into the first fully connected layer of the fully connected neural network for first-layer full connection calculation to generate the first two-dimensional tensor.

The first two-dimensional tensor is sent into the second fully connected layer of the fully connected neural network for second-layer full connection calculation to generate the second two-dimensional tensor.

The second two-dimensional tensor is sent into a third fully connected layer of the fully connected neural network for third-layer full connection calculation to generate a third two-dimensional tensor.

The third two-dimensional tensor is sent into a fourth fully connected layer of the fully connected neural network for second-layer full connection calculation to finally obtain the two-dimensional output tensor.

Here, $B_4=B_3=1$, the number of nodes in the last fully connected layer is $2(W_5=2)$, and the shape of the corresponding final two-dimensional output tensor is specifically $1 \times 2$, which is expressed as two-dimensional output tensor [1,2], and the data of the two-dimensional output tensor [1,2] is (classification reasonable weight data, classification unreasonable weight data).

Step 9, using the normalization processing layer of the artificial intelligence network to perform normalization index calculation on the two-dimensional output tensor to generate a normalization two-dimensional tensor.

Specifically comprising: step 91, sending the two-dimensional output tensor into a normalization processing layer for normalization index calculation of the classification reasonable weight data and the classification unreasonable weight data to generate a classification reasonable probability and a classification unreasonable probability.

Wherein the sum of the classification reasonable probability and the classification unreasonable probability is 1.

Here, the reason why the classification reasonable weight data and the classification unreasonable weight data are subjected to normalization index processing is that through a clear ratio relationship, on the one hand, it can be clear at a glance, and on the other hand, more probability data can be collected for trend analysis. Because the normalization is based on two data, the sum of the two probabilities should be 1.

Step 92, forming a normalization two-dimensional tensor by the classification reasonable probability and the classification unreasonable probability;

Wherein the shape of the normalization two-dimensional tensor is $B_5 \times W_6$, $B_5$ is a two-dimensional parameter of the normalization two-dimensional tensor, and $B_5=B_4=1$, $W_6$ is a one-dimensional parameter of the normalization two-dimensional tensor, and $W_6=W_5=2$, and the normalization two-dimensional tensor comprises two data: classification reasonable probability and classification unreasonable probability.

For example, if the shape of the two-dimensional output tensor is $1\times2$, then $B_5=B_4=1$, $W_6=W_5=2$, the shape of the normalization two-dimensional tensor is also $1\times2$, which is expressed as two-dimensional output tensor [1,2], and the data of the two-dimensional output tensor [1,2] is(classification reasonable probability, classification unreasonable probability).

Step 10, performing classification confirmation according to the normalization two-dimensional tensor to generate confirmation data.

Wherein the confirmation data comprise classification reasonable information and classification unreasonable information.

Specifically comprising: identifying whether the classification reasonable probability is higher than the classification unreasonable probability, and when the classification reasonable probability is higher than the classification unreasonable probability, taking the classification reasonable information as confirmation data, and when the classification reasonable probability is lower than the classification unreasonable probability, taking the classification unreasonable information as confirmation data.

Here, the confirmation data including the classification reasonable information and the classification unreasonable information are information stored in the local storage medium of the equipment.

Here, when the classification reasonable probability is higher than the classification unreasonable probability, it means that the probability of the artificial intelligence network identifying the current scatter diagram as a scatter diagram of a certain type is higher than the probability that it is not of the type. When the classification reasonable probability is lower than the classification unreasonable probability, it means that the probability of the artificial intelligence network identifying the current scatter diagram as a scatter diagram not of a certain type is higher than the probability that it is of the type.

For example, the artificial intelligence network is used to identify an atrial fibrillation scatter diagram, and the data of the two-dimensional output tensor [1,2] is (90%, 10%), where the classification reasonable probability=90%, the classification unreasonable probability=10%, the classification reasonable information is specifically "atrial fibrillation state", and the classification unreasonable information is specifically "non-atrial fibrillation state", then the embodiment of the present invention the confirmation data is set to classification reasonable information ("atrial fibrillation state"). After separate confirmation, the equipment gets the confirmation data of "atrial fibrillation state", and will immediately start a related early warning process.

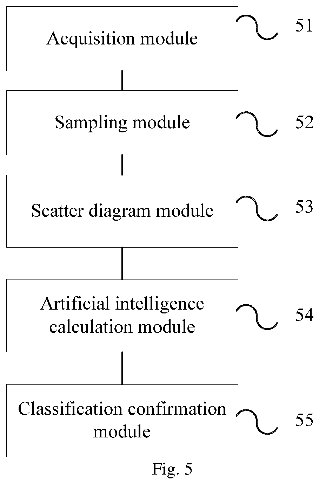

Fig. 5 is a modular diagram schematic of a scatter diagram classification device for a PPG signal provided in a second embodiment of the present invention. The device maybe the terminal equipment or server described in the previous embodiment, or may be a device which enables the terminal equipment or server to implement the method provided in the embodiment of the invention, for example, the device maybe a device or chip system of the above-mentioned terminal equipment or server. As shown in Fig. 5, the device comprises:
an acquisition module 51 for acquiring the PPG signal.

A sampling module 52 for performing signal sampling processing on the PPG signal according to a preset sampling frequency to generate a PPG sampling signal.

A scatter diagram module 53 for performing scatter point two-dimensional coordinate preparation processing according to the PPG sampling signal to generate a scatter point two-dimensional coordinate sequence; performing scatter diagram resolution confirmation processing according to the sampling frequency and a preset maximum value of an inter-beat interval to generate a scatter diagram resolution; performing scatter diagram initialization processing according to the scatter diagram resolution to generate a scatter diagram two-dimensional tensor; and performing scatter point marking on the scatter diagram two-dimensional tensor according to the scatter point two-dimensional coordinate sequence.

An artificial intelligence calculation module 54 for using a convolutional neural network of an artificial intelligence network to perform multilayer convolution pooling calculation on the scatter diagram two-dimensional tensor to generate a four-dimensional output tensor; using a fully connected neural network of the artificial intelligence network to perform multilayer full connection calculation on the four-dimensional output tensor to generate a two-dimensional output tensor; and using a normalization processing layer of the artificial intelligence network to perform normalization index calculation on the two-dimensional output tensor to generate a normalization two-dimensional tensor, the artificial intelligence network comprising the convolutional neural network, the fully connected neural network and the normalization processing layer, and

a classification confirmation module 55 for performing classification confirmation processing according to the normalization two-dimensional tensor to generate confirmation data, the confirmation data comprising classification reasonable information and classification unreasonable information.

The scatter diagram classification device for a PPG signal provided by the embodiment of the present invention can perform the method steps in the above method embodiment, and its implementation principles and technical effects are similar, which will not be repeated here.

It should be understood that the division of different modules of the above device is based on logical functions, and in actual implementation, all or part of the modules can be integrated into a physical entity, or they can be physically separated. These modules can all be implemented in the form of software which can be called by processing elements, or all of them can be implemented in the form of hardware, or some modules are implemented in the form of software which can be called by processing elements, and some modules are implemented in the form of hardware. For example, the acquisition module may be a separate processing element, or may be integrated into a certain chip of the above-mentioned device, or it may be stored in a memory of the above-mentioned device in the form of program code, and called by a certain processing element of the above-mentioned device to implement the functions of the above-mentioned determination module. Other modules are implemented similarly. In addition, all or part of these modules may be integrated or implemented separately. The processing element described here may be an integrated circuit with signal processing capability. In the implementation process, each step of the above method or each module may be realized by an integrated logic circuit of hardware in the processor element or instructions in the form of software.

For example, the above modules may be one or more integrated circuits configured to implement the above method, such as one or more application specific integrated circuits (ASIC), one or more digital signal processors (DSP), one or more field programmable gate arrays (FPGA), etc. For another example, when one of the above modules is implemented in the form of a program code which can be called by a processing element, the processing element may be a general purpose processor, such as a central processing unit (CPU) or other processors which can call the program code. For example, these modules can be integrated and implemented in the form of system-on-a-chip (SOC).

In the above embodiments, the functional units can be implemented in whole or in part by software, hardware, firmware or any combination thereof. When implemented by software, the functional units can be implemented in whole or in part by computer program products. The computer program product comprises one or more computer instructions. When the computer program instructions are loaded and executed on a computer, the flow or function according to the embodiment of the invention is generated in whole or in part. The computer may be a general purpose computer, a special purpose computer, a computer network, or other programmable devices. The computer instructions may be stored in the computer-readable storage medium or transmitted from one computer-readable storage medium to another. For example, the computer instructions may be transmitted from one website, computer, server or data center to another website, computer, server or data center through wired (such as coaxial cable, optical fiber, digital subscriber line (DSL)) or wireless (such as infrared, wireless, Bluetooth and microwave) methods. The computer readable storage medium may be any available medium that can be accessed by a computer, or a data storage device such as server and data center that contains one or more available media integrations. The available medium may be magnetic medium (e.g., floppy disk, hard disk, magnetic tape), optical medium (e.g., DVD), or semiconductor medium (e.g. solid state disk (SSD)).

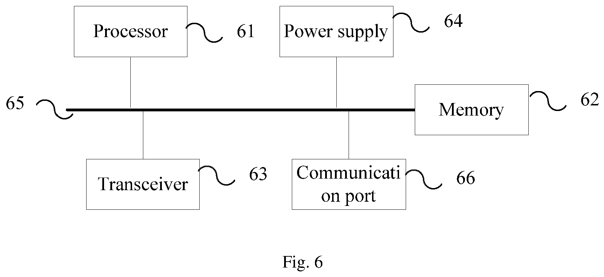

Fig. 6 is a structural diagram schematic of electronic equipment provided by the third embodiment of the present invention. The electronic equipment maybe the aforementioned terminal equipment or server, or maybe the terminal equipment or server connected to the aforementioned terminal equipment or server to realize the method of the embodiment of the invention. As shown in Fig. 6, the electronic equipment may comprise a processor 61 (for example, CPU), a memory 62, and a transceiver 63. The transceiver 63 is coupled to the processor 61, and the processor 61 controls the transceiving action of the transceiver 63. The memory 62 may store various instructions for accomplishing various processing functions and realizing the method and processing procedures provided in the above embodiments of the invention. Preferably, the electronic equipment according to the embodiment of the present invention further comprises a power supply 64, a system bus 65 and a communication port 66. The system bus 65 is configured to realize the communication between components. The communication port 66 is used for communication between the electronic equipment and other peripherals.

The system bus mentioned in Fig. 6 may be a peripheral component interconnect (PCI) bus or an extended industry standard architecture (EISA) bus, etc. The system bus may be divided into an address bus, a data bus and a control bus. For convenience of representation, only a thick line is shown in the figure, but it does not mean that there is only one bus or one type of bus. The communication interface is configured to realize the communication between a database access device and other devices (such as client, read-write library and read-only library). The memory may comprise a random access memory (RAM) or a non-volatile memory, such as at least one disk memory.

The above processor may be a general purpose processor, including a CPU, a network processor (NP), a graphics processing unit (GPU), etc., and may also be a DSP, an ASIC, an FPGA or other programmable logic devices, discrete gate or transistor logic devices, and discrete hardware components.

It should be noted that the embodiment of the present invention also provides a computer-readable storage medium, in which instructions are stored, which, when run on a computer, cause the computer to execute the method and processing procedures provided in the above embodiments.

An embodiment of the invention also provides a chip for running instructions, and the chip is configured to execute the method and processing procedures provided in the above embodiments.

An embodiment of the invention also provides a program product, which comprises a computer program stored in a storage medium. At least one processor may read the computer program from the storage medium, and the at least one processor executes the method and processing procedures provided in the above embodiments.

The embodiment of the invention provides a scatter diagram classification method and device for a PPG signal, electronic equipment, a computer program product and a computer readable storage medium. By extracting inter-beat interval data from PPG signals to generate a scatter diagram, and then introducing the scatter diagram into an artificial intelligence network for confirming the type of the scatter diagram for type confirmation, therefore the scatter diagram generation difficulty is reduced, and the application scenarios of PPG in the field of health monitoring are enriched.

Professionals should further realize that the units and algorithm steps of each example described in connection with the embodiments disclosed herein can be implemented in electronic hardware, computer software or a combination of the two. In order to clearly explain the interchangeability of hardware and software, the components and steps of each example have been generally described according to functions in the above description. Whether these functions are implemented in hardware or software depends on the specific application and design constraints of the technical scheme. Professionals can use different methods to implement the described functions for each specific application, but such implementation should not be considered beyond the scope of the invention.

The steps of a method or algorithm described in connection with the embodiments disclosed herein may be implemented in hardware, a software module executed by a processor, or a combination of the two. The software module can be placed in a random access memory (RAM), memory, read-only memory (ROM), electrically programmable ROM, electrically erasable programmable ROM, register, hard disk, removable magnetic disk, CD-ROM, or any other form of storage medium known in the technical field.

The above-mentioned specific embodiments further explain the purpose, technical scheme and beneficial effects of the invention in detail. It should be understood that the invention is defined by the claims.

## Claims

1. A computer-implemented scatter diagram classification method for a photoplethysmography (PPG) signal, wherein the method comprises:

   acquiring the PPG signal (1);
   performing signal sampling processing on the PPG signal according to a preset sampling frequency to generate a PPG sampling signal (2);
   performing scatter point two-dimensional coordinate preparation processing according to the PPG sampling signal to generate a scatter point two-dimensional coordinate sequence (3);
   **characterized in that** the method further comprises:

   performing scatter diagram resolution confirmation processing according to the sampling frequency and a preset maximum value of an inter-beat interval to generate a scatter diagram resolution (4);
   performing scatter diagram initialization processing according to the scatter diagram resolution to generate a scatter diagram two-dimensional tensor (5);
   performing scatter point marking processing on the scatter diagram two-dimensional tensor according to the scatter point two-dimensional coordinate sequence (6);
   using a convolutional neural network of an artificial intelligence network to perform multi-layer convolution pooling calculation on the scatter diagram two-dimensional tensor to generate a four-dimensional output tensor (7), the artificial intelligence network comprising the convolutional neural network, a fully connected neural network and a normalization processing layer;
   using the fully connected neural network of the artificial intelligence network to perform multi-layer full connection calculation on the four-dimensional output tensor to generate a two-dimensional output tensor (8);

using the normalization processing layer of the artificial intelligence network to perform normalization index calculation on the two-dimensional output tensor to generate a normalization two-dimensional tensor (9); and

performing classification confirmation processing according to the normalization two-dimensional tensor to generate confirmation data, the confirmation data comprising classification reasonable information and classification unreasonable information (10),

wherein performing scatter point two-dimensional coordinate preparation processing according to the PPG sampling signal to generate a scatter point two-dimensional coordinate sequence specifically comprises:

> sequentially extracting time information corresponding to maximum amplitude signal points of signal waveforms from the PPG sampling signal to generate peak time data, and forming a peak time data sequence from the peak time data, the PPG sampling signal comprising a plurality of the signal waveforms, the signal waveform comprising a plurality of signal points, and the number of the peak time data of the peak time data sequence is a first total number n;
>
> taking the peak time data corresponding to the first index i' as current peak time data in the peak time data sequence, performing absolute difference calculation on the current peak time data and a peak time data before the current peak time data to generate a scatter point abscissa $X_i$ corresponding to the second index i, and performing absolute difference calculation on the current peak time data and peak time data after the current peak time data to generate a scatter point ordinate $Y_i$ corresponding to the second index i, scatter point two-dimensional coordinates $XY_i$ being composed of the scatter point abscissa $X_i$ and the scatter point ordinate $Y_i$, the scatter point two-dimensional coordinates $XY_i$ being $(X_i, Y_i)$, the first index i' being an index number of the peak time data, and the value range of the first index i' is 2 to n-1, the second index i being an index number of the scatter point two-dimensional coordinates $XY_i$, i=i'-1, and the value range of the second index i is 1 to n-2;

n-2 scatter point two-dimensional coordinates $XY_i$ forming the scatter point two-dimensional coordinate sequence, wherein the scatter point two-dimensional coordinate sequence being $(XY_1,...XY_i,...XY_{n-2})$; and

only keeping one of the plurality of identical scatter point two-dimensional coordinates $XY_i$ in the scatter point two-dimensional coordinate sequence, deleting the scatter point two-dimensional coordinate $XY_i$ of which the scatter point abscissa $X_i$ exceeds the maximum value of inter-beat interval, and deleting the scatter point two-dimensional coordinate $XY_i$ of which the scatter point ordinate $Y_i$ exceeds the maximum value of the inter-beat interval.

2. The scatter diagram classification method for a PPG signal according to claim 1, **characterized in that** performing scatter diagram resolution confirmation processing according to the sampling frequency and a preset maximum value of inter-beat interval to generate a scatter diagram resolution specifically comprises:

> performing maximum pixel number calculation according to the sampling frequency and the maximum value of the inter-beat interval to generate a maximum number of pixels a, a=sampling frequency * maximum value of inter-beat interval; and
>
> the number of horizontal pixels X of the scatter diagram resolution is set to the maximum number of pixels a, and the number of vertical pixels Y of the scatter diagram resolution is set to the maximum number of pixels a, and the scatter diagram resolution = X*Y = a*a.

3. The scatter diagram classification method for a PPG signal according to claim 2, **characterized in that** performing scatter diagram initialization processing according to the scatter diagram resolution to generate a scatter diagram two-dimensional tensor specifically comprises:

setting the scatter diagram two-dimensional tensor according to the scatter diagram resolution, the shape of the scatter diagram two-dimensional tensor being $H_i \times W_1$, the $H_1$ being a two-dimensional parameter of the scatter diagram two-dimensional tensor, and $H_1=Y=a$, the $W_1$ being a one-dimensional parameter of the scatter diagram two-dimensional tensor, and $W_1=X=a$, the scatter diagram two-dimensional tensor comprising $H_1*W_1$ pixel data $D_{S,Z}$, and the value of the pixel data $D_{S,Z}$ being a preset first pixel value, the S being a horizontal subscript of the pixel data $D_{S,Z}$, the value range of S is 1 to $W_1$, Z being a vertical subscript of the pixel data $D_{S,Z}$, and the value range of Z is 1 to $H_1$.

4. The scatter diagram classification method for a PPG signal according to claim 3, **characterized in that** performing scatter point marking processing on the scatter diagram two-dimensional tensor according to the scatter point two-dimensional coordinate sequence specifically comprises:
in the scatter diagram two-dimensional tensor, the value of the pixel data $D_{S,Z}$ matched with the scatter point two-dimensional coordinates $XY_i$ of the scatter point two-dimensional coordinate sequence is set to the preset second pixel value, the horizontal subscript S of the pixel data $D_{S,Z}$ being the same as the scatter point abscissa $X_i$ of the scatter point two-dimensional coordinates $XY_i$, the vertical subscript Z of the pixel data $D_{S,Z}$ being the same as the scatter point ordinate $Y_i$ of the scatter point two-dimensional coordinates $XY_i$.

5. The scatter diagram classification method for a PPG signal according to claim 3, **characterized in that** using a convolutional neural network of an artificial intelligence network to perform multi-layer convolution pooling calculation on the scatter diagram two-dimensional tensor to generate a four-dimensional output tensor specifically comprises:

according to a four-dimensional tensor input data format of the convolutional neural network, raising the shape of the scatter diagram two-dimensional tensor from a two-dimensional tensor shape to a four-dimensional tensor shape to generate a four-dimensional input tensor, the shape of the four-dimensional input tensor being $B_1 \times H_2 \times W_2 \times C_1$, the $B_1$ being a four-dimensional parameter of the four-dimensional input tensor, and $B_1=1$, the $H_2$ being a three-dimensional parameter of the four-dimensional input tensor, and $H_2=H_1$, the $W_2$ being a two-dimensional parameter of the four-dimensional input tensor, and $W_2=W_1$, the $C_1$ being a one-dimensional parameter of the four-dimensional input tensor, and $C_1=1$; and
sending the four-dimensional input tensor into a first convolutional network layer of the convolutional neural network for first-layer convolution pooling calculation to generate a first four-dimensional tensor, then sending the first four-dimensional tensor into a second convolutional network layer of the convolutional neural network for second-layer convolution pooling calculation to generate a second four-dimensional tensor, until finally, sending a penultimate four-dimensional tensor into a last convolutional network layer of the convolutional neural network for last-layer convolution pooling calculation to generate the four-dimensional output tensor, the convolutional neural network comprising a plurality of convolutional network layers, the convolutional network layer comprising a convolution layer and a pooling layer, the shape of the four-dimensional output tensor being $B_2 \times H_3 \times W_3 \times C_2$, the $B_2$ being a four-dimensional parameter of the four-dimensional output tensor, and $B_2=B_1=1$, the $H_3$ being a three-dimensional parameter of the four-dimensional output tensor, the $W_3$ being a two-dimensional parameter of the four-dimensional output tensor, and the $C_2$ being a one-dimensional parameter of the four-dimensional output tensor,
wherein sending the four-dimensional input tensor into a first convolutional network layer of the convolutional neural network for first-layer convolution pooling calculation to generate a first four-dimensional tensor is to send the four-dimensional input tensor to a first convolution layer of the first convolutional network layer for first convolution calculation to generate a first convolution four-dimensional tensor, and then send the first convolution four-dimensional tensor into a first pooling layer of the first convolutional network layer for first pooling calculation to generate the first four-dimensional tensor.

6. The scatter diagram classification method for a PPG signal according to claim 5, **characterized in that** using the fully connected neural network of the artificial intelligence network to perform multi-layer full connection calculation on the four-dimensional output tensor to generate a two-dimensional output tensor specifically comprises:

according to a two-dimensional tensor input data format of the fully connected neural network, reducing the shape of the four-dimensional output tensor from a four-dimensional tensor shape to a two-dimensional tensor shape to generate a two-dimensional input tensor, the shape of the two-dimensional input tensor being $B_3 \times W_4$, the $B_3$ being a two-dimensional parameter of the two-dimensional input tensor, and $B_3=B_2=1$, the $W_4$ being a one-dimensional parameter of the two-dimensional input tensor, and $W_4= H_3*W_3*C_2$; and
sending the two-dimensional input tensor into a first fully connected layer of the fully connected neural network for first-layer full connection calculation to generate a first two-dimensional tensor, then sending the first two-dimensional tensor to a second fully connected layer of the fully connected neural network for second-layer full connection calculation to generate a second two-dimensional tensor, until finally sending a penultimate two-dimensional tensor into a last fully connected layer of the fully connected neural network for last-layer full connection calculation to generate the two-dimensional output tensor, the fully connected neural network comprising a plurality of fully connected layers, the shape of the two-dimensional output tensor being $B_4 \times W_5$, the $B_4$ being a two-dimensional parameter of the two-dimensional output tensor, and $B_4=B_3=1$, the $W_5$ being a one-dimensional parameter of the two-dimensional output tensor, and $W_5=2$, and the two-dimensional output tensor comprising two data: classification reasonable weight data and classification unreasonable weight data.

7. The scatter diagram classification method for a PPG signal according to claim 6, **characterized in that** using the normalization processing layer of the artificial intelligence network to perform normalization index calculation on the two-dimensional output tensor to generate a normalization two-dimensional tensor specifically comprises:

sending the two-dimensional output tensor into the normalization processing layer for normalization index calculation of the classification reasonable weight data and the classification unreasonable weight data to generate a classification reasonable probability and a classification unreasonable probability, the sum of the classification reasonable probability and the classification unreasonable probability being 1; and

forming the normalization two-dimensional tensor by the classification reasonable probability and the classification unreasonable probability, the shape of the normalization two-dimensional tensor being $B_5 \times W_6$, the $B_5$ being a two-dimensional parameter of the normalization two-dimensional tensor, and $B_5=B_4=1$, the $W_6$ being a one-dimensional parameter of the normalization two-dimensional tensor, and $W_6=W_5=2$, and the normalization two-dimensional tensor comprising two data: the classification reasonable probability and the classification unreasonable probability.

8. The scatter diagram classification method for a PPG signal according to claim 7, **characterized in that** performing classification confirmation processing according to the normalization two-dimensional tensor to generate confirmation data (the confirmation data comprising classification reasonable information and classification unreasonable information) specifically comprises:

taking the classification reasonable information as the confirmation data when the classification reasonable probability is higher than the classification unreasonable probability; and

taking the classification unreasonable information as the confirmation data when the classification reasonable probability is lower than the classification unreasonable probability.

9. A scatter diagram classification device for a photoplethysmography (PPG) signal, **characterized in that**, the device comprising:

an acquisition module (51) for acquiring the PPG signal;

a sampling module (52) for performing signal sampling processing on the PPG signal according to a preset sampling frequency to generate a PPG sampling signal;

a scatter diagram module (53) for performing scatter point two-dimensional coordinate preparation processing according to the PPG sampling signal to generate a scatter point two-dimensional coordinate sequence; performing scatter diagram resolution confirmation processing according to the sampling frequency and a preset maximum value of an inter-beat interval to generate a scatter diagram resolution; performing scatter diagram initialization processing according to the scatter diagram resolution to generate a scatter diagram two-dimensional tensor; and performing scatter point marking processing on the scatter diagram two-dimensional tensor according to the scatter point two-dimensional coordinate sequence;

an artificial intelligence calculation module (54) for using a convolutional neural network of an artificial intelligence network to perform multilayer convolution pooling calculation on the scatter diagram two-dimensional tensor to generate a four-dimensional output tensor; using a fully connected neural network of the artificial intelligence network to perform multi-layer full connection calculation on the four-dimensional output tensor to generate a two-dimensional output tensor; and using a normalization processing layer of the artificial intelligence network to perform normalization index calculation on the two-dimensional output tensor to generate a normalization two-dimensional tensor, the artificial intelligence network comprising the convolutional neural network, the fully connected neural network and the normalization processing layer; and

a classification confirmation module (55) for performing classification confirmation processing according to the normalization two-dimensional tensor to generate confirmation data, the confirmation data comprising classification reasonable information and classification unreasonable information.

10. A computer program product comprising a computer program code that, when executed by a computer, causes the computer to execute the method according to any one of claims 1-8.

11. A computer-readable storage medium, which stores computer instructions that, when executed by a computer, cause the computer to execute the method according to any one of claims 1-8.

**Patentansprüche**

1. Computerimplementiertes Verfahren zur Klassifizierung von Streudiagrammen für ein Photoplethysmografie- (PPG) Signal, aufweisend:

   Erfassen des PPG-Signals (1),
   Durchführen einer Signalprobenverarbeitung auf dem PPG-Signal entsprechend einer voreingestellten Probenfrequenz, um ein PPG-Probensignal (2) zu erzeugen,
   Durchführen einer Vorbereitung einer zweidimensionalen Koordinatenstreupunktverarbeitung entsprechend dem PPG-Probensignal, um eine Sequenz (3) von zweidimensionalen Koordinatenstreupunkten zu erzeugen;
   **dadurch gekennzeichnet, dass** das Verfahren ferner umfasst:

   Durchführen einer Bestätigungsverarbeitung einer Streudiagrammauflösung entsprechend der Probenfrequenz und einem voreingestellten Maximalwert eines Inter-Beat-Intervalls, um eine Streudiagrammauflösung (4) zu erzeugen;
   Durchführen einer Verarbeitung einer Streudiagramminitialisierung entsprechend der Streudiagrammauflösung, um einen zweidimensionalen Streudiagrammtensor (5) zu erzeugen;
   Durchführen einer Verarbeitung einer Streupunktmarkierung auf dem zweidimensionalen Streudiagrammtensor entsprechend der Sequenz (6) von zweidimensionalen Koordinatenstreupunkten;
   Verwenden eines konvolutionalen neuronalen Netzwerks eines künstlichen Intelligenznetzwerks, um eine mehrschichtige Konvolutions-Pooling-Berechnung auf dem zweidimensionalen Streudiagrammtensor durchzuführen, um einen vierdimensionalen Ausgangstensor (7) zu erzeugen, wobei das künstliche Intelligenznetzwerk das konvolutionale neuronale Netzwerk, ein vollständig verbundenes neuronales Netzwerk und einen Normalisierungs- Verarbeitungslayer umfasst;
Verwenden des vollständig verbundenen neuronalen Netzwerks des künstlichen Intelligenznetzwerks, um eine Berechnung einer vollständigen mehrschichtigen Verbindung auf dem vierdimensionalen Ausgangstensor durchzuführen, um einen zweidimensionalen Ausgangstensor (8) zu erzeugen;
Verwenden des Normalisierungs-Verarbeitungslayers des künstlichen Intelligenznetzwerks, um eine Berechnung eines Normalisierungsindex auf dem zweidimensionalen Ausgangstensor durchzuführen, um einen zweidimensionalen Normalisierungstensor (9) zu erzeugen; und
Durchführen einer Bestätigungsverarbeitung einer Klassifikation entsprechend dem zweidimensionalen Normalisierungstensor, um Bestätigungsdaten zu erzeugen, wobei die Bestätigungsdaten annehmbare Klassifikationsinformationen und unannehmbare Klassifikationsinformationen (10) umfassen,
wobei ein Durchführen einer Vorbereitung einer zweidimensionalen Koordinatenstreupunktverarbeitung entsprechend dem PPG-Probensignal durchgeführt wird, um eine Sequenz von zweidimensionalen Koordinatenstreupunkten zu erzeugen, spezifisch umfassend:

   nacheinander ein Extrahieren von Zeitinformationen entsprechend dem Amplitudenmaximum der Signalpunkte der Signalwellenformen von dem PPG-Probensignal, um Daten über Scheitelwertzeitpunkte zu erzeugen, und um eine Sequenz von Daten über Scheitelwertzeitpunkte aus den Daten über Scheitelwertzeitpunkte zu bilden, wobei das PPG-Probensignal mehrere Signalwellenformen umfasst, wobei die Signalwellenform mehrere Signalpunkte umfasst, und wobei die Anzahl der Daten über Scheitelwertzeitpunkte der Sequenz von Daten über Scheitelwertzeitpunkte eine erste ganze Zahl n ist;
   Aufnehmen der Daten über Scheitelwertzeitpunkte entsprechend dem ersten Index i' als laufende Daten über Scheitelwertzeitpunkte in die Sequenz der Daten über Scheitelwertzeitpunkte, Durchführen einer Berechnung einer absoluten Differenz zwischen den laufenden Daten über Scheitelwertzeitpunkte und den Daten über Scheitelwertzeitpunkte zeitlich vor den laufenden Daten , um eine Streupunktabszisse $X_i$ entsprechend dem zweiten Index i zu erzeugen, und Durchführen einer Berechnung einer absoluten Differenz zwischen den laufenden Daten über Scheitelwertzeitpunkte und den Daten über Scheitelwertzeitpunkte zeitlich nach den laufenden Daten, um eine Streupunktordinate $Y_i$ entsprechend dem zweiten Index i zu erzeugen, wobei die zweidimensionalen Koordinatenstreupunkte $XY_i$ aus der Streupunktabszisse $X_i$ und der Streupunktordinate $Y_i$ zusammengesetzt werden, wobei die zweidimensionalen Koordinatenstreupunkte $XY_i$ $(X_iY_i)$ sind, wobei der erste Index i' eine Indexnummer der Daten über Scheitelwertzeitpunkte ist, und wobei sich der Wertebereich des ersten Index i' von 2 bis n-1 erstreckt, wobei der zweite Index i eine Indexnummer der zweidimensionalen Koordinatenstreupunkte $XY_i$ ist mit i=i'-1, und wobei sich der Wertebereich des zweiten Index i von 1 bis n-2 erstreckt; wobei n-2 zweidimensionale Koordinatenstreupunkte $XY_i$ die Sequenz der zweidimensionalen Koordinatenstreupunkte bilden, wobei die Sequenz der zweidimensionalen Koordinatenstreupunkte aus $(XY_1,...XY_i,... XY_{n-2})$ ist; und Behalten nur eines von mehreren identischen zweidimensionalen Koordinatenstreupunkten $XY_i$ in der Sequenz der zweidimensionalen Koordinatenstreupunkte, Löschen derjenigen zweidimensionalen Koordinatenstreupunkte $XY_i$, von denen die Streupunktabszisse $X_i$ den Maximalwert eines Inter-Beat-Intervalls überschreitet, und Löschen derjenigen zweidimensionalen Koordinatenstreupunkte $XY_i$, von denen die Streupunktordinate $Y_i$ den Maximalwert des Inter-Beat-Intervalls überschreitet.

2. Verfahren zur Klassifizierung von Streudiagrammen für ein PPG-Signal nach Anspruch 1, **dadurch gekennzeichnet, dass** ein Durchführen einer Bestätigungsverarbeitung einer Streudiagrammauflösung entsprechend der Probenfrequenz und einem voreingestellten Maximalwert eines Inter-Beat-Intervalls, um eine Streudiagrammauflösung zu erzeugen, spezifisch umfasst:

Durchführen einer Berechnung einer maximalen Pixelanzahl entsprechend der Probenfrequenz und dem Maximalwert des Inter-Beat-Intervalls, um eine maximale Pixelanzahl a zu erzeugen, wobei a=Probenfrequenz* Maximalwert eines Inter-Beat-Intervalls; und wobei die Anzahl der horizontalen Pixel X der Streudiagrammauflösung auf die maximale Pixelanzahl a eingestellt ist, und wobei die Anzahl der vertikalen Pixel Y der Streudiagrammauflösung auf die maximale Pixelanzahl a eingestellt ist, und wobei gilt: Streudiagrammauflösung = X*Y = a*a.

3. Verfahren zur Klassifizierung von Streudiagrammen für ein PPG-Signal nach Anspruch 2, **dadurch gekennzeichnet, dass** ein Durchführen einer Verarbeitung einer Streudiagramminitialisierung entsprechend der Streudiagrammauflösung, um einen zweidimensionalen Streudiagrammtensor zu erzeugen, spezifisch umfasst:
Einstellen des zweidimensionalen Streudiagrammtensors entsprechend der Streudiagrammauflösung, wobei die Form des zweidimensionalen Streudiagrammtensors $H_1 \times W_1$ ist, wobei $H_1$ ein zweidimensionaler Parameter des zweidimensionalen Streudiagrammtensors ist, und wobei $H_1$=Y=a ist, wobei $W_1$ ein eindimensionaler Parameter des zweidimensionalen Streudiagrammtensors ist, und wobei $W_1$=X=a ist, wobei der zweidimensionale Streudiagrammtensor $H_1*W_1$-Pixeldaten $D_{S,Z}$ umfasst, und wobei der Wert der Pixeldaten $D_{S,Z}$ ein voreingestellter erster Pixelwert ist, wobei S ein horizontaler tiefgestellter Index der Pixeldaten $D_{S,Z}$ ist, wobei der Wertebereich von S von 1 bis $W_1$ reicht, wobei Z ein vertikaler tiefgestellter Index der Pixeldaten $D_{S,Z}$ ist, und wobei der Wertebereich von Z von 1 bis $H_1$ reicht.

4. Verfahren zur Klassifizierung von Streudiagrammen für ein PPG-Signal nach Anspruch 3, **dadurch gekennzeichnet, dass** ein Durchführen einer Verarbeitung einer Streupunktmarkierung auf dem zweidimensionalen Streudiagrammtensor entsprechend der Folge der zweidimensionalen Koordinatenstreupunkte spezifisch umfasst:
in dem zweidimensionalen Streudiagrammtensor wird der Wert der Pixeldaten $D_{S,Z}$, abgestimmt mit den zweidimensionalen Koordinatenstreupunkten $XY_i$ der Folge der zweidimensionalen Koordinatenstreupunkte, auf den voreingestellten zweiten Pixelwert eingestellt, wobei der horizontale tiefgestellte Index S der Pixeldaten $D_{S,Z}$ derselbe ist wie die Streupunktabszisse $X_i$ der zweidimensionalen Koordinatenstreupunkte $XY_i$, wobei der vertikale tiefgestellte Index Z der Pixeldaten $D_{S,Z}$ derselbe ist wie die Streupunktordinate $Y_i$ der zweidimensionalen Koordinatenstreupunkte $XY_i$.

**5.** Verfahren zur Klassifizierung von Streudiagrammen für ein PPG-Signal nach Anspruch 3, **dadurch gekennzeichnet, dass** ein Verwenden eines konvolutionalen neuronalen Netzwerks eines künstlichen Intelligenznetzwerks, um eine mehrschichtige Konvolutions-Pooling-Berechnung auf dem zweidimensionalen Streudiagrammtensor durchzuführen, um einen vierdimensionalen Ausgangstensor zu erzeugen, spezifisch umfasst:

entsprechend einem Eingangsdatenformat eines vierdimensionalen Tensors des konvolutionalen neuronalen Netzwerks ein wird die Form des zweidimensionalen Streudiagrammtensors von einer zweidimensionalen Tensorform auf eine vierdimensionale Tensorform erhöht, um einen vierdimensionalen Eingangstensor zu erzeugen, wobei die Form des vierdimensionalen Eingangstensors $B_1 x H_2 x W_2 x C_1$ ist, wobei $B_1$ ein vierdimensionaler Parameter des vierdimensionalen Eingangstensors ist, und wobei $B_1=1$ ist, wobei $H_2$ ein dreidimensionaler Parameter des vierdimensionalen Eingangstensors ist, und wobei $H_2=H_1$ ist, wobei $W_2$ ein zweidimensionaler Parameter des vierdimensionalen Eingangstensors ist, und wobei $W_2=W_1$ ist, wobei $C_1$ ein eindimensionaler Parameter des vierdimensionalen Eingangstensors ist, und wobei $C_1=1$ ist; und

Senden des vierdimensionalen Eingangstensors in einen ersten konvolutionalen Netzwerklayer des konvolutionalen neuronalen Netzwerks für eine Konvolutions-Pooling-Berechnung eines ersten Layers, um einen ersten vierdimensionalen Tensor zu erzeugen, dann Senden des ersten vierdimensionalen Tensors in einen zweiten konvolutionalen Netzwerklayer des konvolutionalen neuronalen Netzwerks für eine Konvolutions-Pooling-Berechnung eines zweiten Layers, um einen zweiten vierdimensionalen Tensor zu erzeugen, solange schließlich bis zum Senden eines vorletzten vierdimensionalen Tensors in einen letzte konvolutionale Netzwerklayer des konvolutionalen neuronalen Netzwerks für eine Konvolutions-Pooling-Berechnung für einen letzten Layer, um den vierdimensionalen Ausgangstensor zu erzeugen, wobei das konvolutionale neuronale Netzwerk mehrere der konvolutionalen Netzwerklayer umfasst, wobei der konvolutionale Netzwerklayer einen Konvolutionslayer und einen Poolinglayer umfasst, wobei die Form des vierdimensionalen Ausgangstensors $B_2 x H_3 x W_3 x C_2$ ist, wobei $B_2$ ein vierdimensionaler Parameter des vierdimensionalen Ausgangstensors ist, und wobei $B_2=B_1=1$ ist, wobei $H_3$ ein dreidimensionaler Parameter des vierdimensionalen Ausgangstensors ist, wobei $W_3$ ein zweidimensionaler Parameter des vierdimensionalen Ausgangstensors ist, und wobei $C_2$ ein eindimensionaler Parameter des vierdimensionalen Ausgangstensors ist,

wobei das Senden des vierdimensionalen Eingangstensors in einen ersten konvolutionalen Netzwerklayer des konvolutionalen neuronalen Netzwerks für eine Konvolutions-Pooling-Berechnung eines ersten Layers, um einen ersten vierdimensionalen Tensor zu erzeugen, derart ausgestaltet ist, um den vierdimensionalen Eingangstensor zu einem ersten konvolutionalen Layer der ersten konvolutionalen Netzwerkschicht für eine erste Konvolutions-Berechnung zu senden, um einen ersten vierdimensionalen Konvolutiontensor zu erzeugen, und um dann den ersten vierdimensionalen Konvolutiontensor in einen ersten Poolinglayer des ersten konvolutionalen Netzwerklayers für eine erste Poolingberechnung zu senden, um den ersten vierdimensionalen Tensor zu erzeugen.

**6.** Verfahren zur Klassifizierung von Streudiagrammen für ein PPG-Signal nach Anspruch 5, **dadurch gekennzeichnet, dass** ein Verwenden des vollständig verbundenen neuronalen Netzwerks des künstlichen Intelligenznetzwerks, um eine mehrschichtige Konvolutionsberechnung auf dem vierdimensionalen Ausgangstensor durchzuführen, um einen zweidimensionalen Ausgangstensor zu erzeugen, spezifisch umfasst:

entsprechend einem Eingangsdatenformat eines zweidimensionalen Tensors des vollständig verbundenen neuronalen Netzwerks wird die Form des vierdimensionalen Ausgangstensors von einer vierdimensionalen Tensorform auf eine zweidimensionale Tensorform reduziert, um einen zweidimensionalen Eingangstensor zu erzeugen, wobei die Form des zweidimensionalen Eingangstensors $B_3 x W_4$ ist, wobei $B_3$ ein zweidimensionaler Parameter des zweidimensionalen Eingangstensors ist, und wobei $B_3=B_2=1$ ist, wobei $W_4$ ein eindimensionaler Parameter des zweidimensionalen Eingangstensors ist, und wobei $W_4=H_3{}^*W_3{}^*C_2$ ist; und

Senden des zweidimensionalen Eingangstensors in einen ersten vollständig verbundenen Layer des vollständig verbundenen neuronalen Netzwerks für eine Berechnung der vollständigen Verbindung des ersten Layers, um einen ersten zweidimensionalen Tensor zu erzeugen, dann Senden des ersten zweidimensionalen Tensors zu einem zweiten vollständig verbundenen Layer des vollständig verbundenen neuronalen Netzwerks für eine Berechnung der vollständigen Verbindung des zweiten Layers, um einen zweiten zweidimensionalen Tensor zu erzeugen, solange schließlich bis zum Senden eines vorletzten zweidimensionalen Tensors in einen letzten vollständig verbundenen Layer des vollständig verbundenen neuronalen Netzwerks für eine Berechnung der vollständigen Verbindung des letzten Layers, um den zweidimensionalen Ausgangstensor zu erzeugen, wobei das vollständig verbundene neuronale Netzwerk mehrere der vollständig verbundenen Netzwerklayer umfasst, wobei die Form des zweidimensionalen Ausgangstensors $B_4 x W_5$ ist, wobei $B_4$ ein zweidimensionaler Parameter des zweidimensionalen Ausgangstensors ist, und wobei $B_4=B_3=1$ ist, wobei $W_5$ ein eindimensionaler Parameter des zweidimensionalen Ausgangstensors ist, und wobei $W_5=2$ ist, und wobei der zweidimensionale Ausgangstensor zwei Daten umfasst: annehmbare Klassifikationsgewichtsdaten und unannehmbare Klassifikationsgewichtsdaten.

7. Verfahren zur Klassifizierung von Streudiagrammen für ein PPG-Signal nach Anspruch 6, **dadurch gekennzeichnet, dass** ein Verwenden des Normalisierungs-Verarbeitungslayers des künstlichen Intelligenznetzwerks, um eine Berechnung eines Normalisierungsindex auf dem zweidimensionalen Ausgangstensor durchzuführen, um einen zweidimensionalen Normalisierungstensor zu erzeugen, spezifisch umfasst:

Senden des zweidimensionalen Ausgangstensors in den Normalisierungs-Verarbeitungslayer für eine Berechnung eines Normalisierungsindex der annehmbaren Klassifikationsgewichtsdaten und der unannehmbaren Klassifikationsgewichtsdaten, um eine Klassifikation einer annehmbaren Wahrscheinlichkeit und eine Klassifikation einer unannehmbaren Wahrscheinlichkeit zu erzeugen, wobei die Summe aus der Klassifikation der annehmbaren Wahrscheinlichkeit und aus der Klassifikation der unannehmbaren Wahrscheinlichkeit gleich 1 ist; und
Bilden des zweidimensionalen Normalisierungstensors durch die Klassifikation der annehmbaren Wahrscheinlichkeit und durch die Klassifikation der unannehmbaren Wahrscheinlichkeit, wobei die Form des zweidimensionalen Normalisierungstensors $B_5 x W_6$ ist, wobei $B_5$ ein zweidimensionaler Parameter des zweidimensionalen Normalisierungstensors ist, und wobei $B_5=B_4=1$ ist, wobei $W_6$ ein eindimensionaler Parameter des zweidimensionalen Normalisierungstensors ist, und wobei $W_6=W_5=2$ ist, und wobei der zweidimensionale Normalisierungstensor zwei Daten umfasst: die Klassifikation einer annehmbaren Wahrscheinlichkeit und die Klassifikation einer unannehmbaren Wahrscheinlichkeit.

8. Verfahren zur Klassifizierung von Streudiagrammen für ein PPG-Signal nach Anspruch 7, **dadurch gekennzeichnet, dass** ein Durchführen einer Bestätigungsverarbeitung einer Klassifikation entsprechend dem zweidimensionalen Normalisierungstensor, um Bestätigungsdaten zu erzeugen (die Bestätigungsdaten umfassen annehmbare Klassifikationsinformationen und unannehmbare Klassifikationsinformationen), spezifisch umfasst:

ein Aufnehmen der annehmbaren Klassifikationsinformationen als die Bestätigungsdaten dann, wenn die Klassifikation der annehmbaren Wahrscheinlichkeit größer als die der Klassifikation einer unannehmbaren Wahrscheinlichkeit ist; und
ein Aufnehmen der unannehmbaren Klassifikationsinformationen als die Bestätigungsdaten dann, wenn die Klassifikation der annehmbaren Wahrscheinlichkeit kleiner als die der Klassifikation der unannehmbaren Wahrscheinlichkeit ist.

9. Vorrichtung zur Klassifizierung von Streudiagrammen für ein Photoplethysmografie (PPG)-Signal, **dadurch gekennzeichnet**, aufweisend:

ein Erfassungsmodul (51) zum Erfassen des PPG-Signals;
ein Probenmodul (52) zum Durchführen einer Signalprobenverarbeitung auf dem PPG-Signal entsprechend einer voreingestellten Probenfrequenz, um ein PPG-Probensignal zu erzeugen;
ein Streudiagrammmodul (53) zum Durchführen einer Vorbereitung einer zweidimensionalen Koordinatenstreupunktverarbeitung entsprechend dem PPG-Probensignal, um eine Sequenz von zweidimensionalen Koordinatenstreupunkten zu erzeugen, und zum Durchführen einer Bestätigungsverarbeitung einer Streudiagrammauflösung entsprechend der Probenfrequenz und einem voreingestellten Maximalwert eines Inter-Beat-Intervalls, um eine Streudiagrammauflösung zu erzeugen, und zum Durchführen einer Verarbeitung einer Streudiagramminitialisierung entsprechend der Streudiagrammauflösung, um einen zweidimensionalen Streudiagrammtensor zu erzeugen, und zum Durchführen einer Verarbeitung einer Streupunktmarkierung auf dem zweidimensionalen Streudiagrammtensor entsprechend der Sequenz von zweidimensionalen Koordinatenstreupunkten,

ein Berechnungsmodul (54) einer künstlichen Intelligenz, um ein konvolutionales neuronales Netzwerk eines künstlichen Intelligenznetzwerks dazu zu verwenden, um eine mehrschichtige Konvolutions-Pooling-Berechnung auf dem zweidimensionalen Streudiagrammtensor durchzuführen, um einen vierdimensionalen Ausgangstensor zu erzeugen, und um ein vollständig verbundenes neuronales Netzwerk des künstlichen Intelligenznetzwerks zu verwenden, um eine Berechnung einer vollständigen mehrschichtigen Verbindung auf dem vierdimensionalen Ausgangstensor durchzuführen, um einen zweidimensionalen Ausgangstensor zu erzeugen, und um einen Normalisierungs-Verarbeitungslayer des künstlichen Intelligenznetzwerks zu verwenden, um eine Berechnung eines Normalisierungsindex auf dem zweidimensionalen Ausgangstensor durchzuführen, um einen zweidimensionalen Normalisierungstensor zu erzeugen, wobei das künstliche Intelligenznetzwerk das konvolutionale neuronale Netzwerk, das vollständig verbundene neuronale Netzwerk und den Normalisierungs-Verarbeitungslayer umfasst; und

ein Klassifikationsbestätigungsmodul (55), um eine Bestätigungsverarbeitung einer Klassifikation entsprechend dem zweidimensionalen Normalisierungstensor durchführen, um Bestätigungsdaten zu erzeugen, wobei die Bestätigungsdaten annehmbare Klassifikationsinformationen und unannehmbare Klassifikationsinformationen umfassen.

10. Computerprogrammprodukt, das einen Computerprogrammcode umfasst, der, wenn er von einem Computer ausgeführt wird, den Computer veranlasst, das Verfahren nach einem der Ansprüche 1 bis 8 auszuführen.

11. Computerlesbares Speichermedium, das Computeranweisungen speichert, die, wenn sie von einem Computer ausgeführt werden, den Computer veranlassen, das Verfahren nach einem der Ansprüche 1 bis 8 auszuführen.

## Revendications

1. Procédé informatisé de classification par diagramme de dispersion pour un signal de photopléthysmographie (PPG),
le procédé comprenant :

l'acquisition du signal PPG (1) ;
la réalisation d'un traitement d'échantillonnage de signal sur le signal PPG selon une fréquence d'échantillonnage prédéfinie pour générer un signal d'échantillonnage PPG (2) ;
la réalisation d'un traitement de préparation de coordonnées bidimensionnelles de points de dispersion en fonction du signal d'échantillonnage PPG pour générer une séquence de coordonnées bidimensionnelles de points de dispersion (3) ;
**caractérisé en ce que** le procédé comprend en outre :

la réalisation d'un traitement de confirmation de résolution de diagramme de dispersion en fonction de la fréquence d'échantillonnage et d'une valeur maximale prédéfinie d'un intervalle inter-battements pour générer une résolution de diagramme de dispersion (4) ;
la réalisation d'un traitement d'initialisation de diagramme de dispersion selon la résolution du diagramme de dispersion pour générer un tenseur bidimensionnel de diagramme de dispersion (5) ;
la réalisation d'un traitement de marquage des points de dispersion sur le tenseur bidimensionnel du diagramme de dispersion en fonction de la séquence de coordonnées bidimensionnelles des points de dispersion (6) ;
l'utilisation d'un réseau neuronal convolutif d'un réseau d'intelligence artificielle pour effectuer un calcul de regroupement convolutif multicouche sur le tenseur bidimensionnel du diagramme de dispersion pour générer un tenseur de sortie quadridimensionnel (7), le réseau d'intelligence artificielle comprenant le réseau neuronal convolutif, un réseau de traitement neuronal entièrement connecté et une couche de traitement de normalisation ;
l'utilisation du réseau neuronal entièrement connecté du réseau d'intelligence artificielle pour effectuer un calcul de connexion complète multicouche sur le tenseur de sortie quadridimensionnel afin de générer un tenseur de sortie bidimensionnel (8) ;
l'utilisation de la couche de traitement de normalisation du réseau d'intelligence artificielle pour effectuer un calcul d'indice de normalisation sur le tenseur de sortie bidimensionnel afin de générer un tenseur bidimensionnel de normalisation (9) ; et
la réalisation d'un traitement de confirmation de classification selon le tenseur bidimensionnel de normalisation pour générer des données de confirmation, les données de confirmation comprenant des informations raisonnables de classification et des informations déraisonnables de classification (10),

l'exécution d'un traitement de préparation de coordonnées bidimensionnelles de points de dispersion selon le signal d'échantillonnage PPG pour générer une séquence de coordonnées bidimensionnelles de points de dispersion comprenant spécifiquement :

l'extraction séquentielle des informations temporelles correspondant aux points de signal d'amplitude maximale des formes d'onde de signal du signal d'échantillonnage PPG pour générer des données temporelles de crête, et former une séquence de données temporelles de crête à partir des données temporelles de crête, le signal d'échantillonnage PPG comprenant une pluralité de formes d'onde de signal, la forme d'onde de signal comprenant une pluralité de points de signal, et le nombre de données temporelles de crête de la séquence de données temporelles de crête étant un premier nombre total n ; l'adoption des données temporelles de crête correspondant au premier indice i' comme données temporelles de crête actuelles dans la séquence de données temporelles de crête, la réalisation d'un calcul de différence absolue sur les données temporelles de crête actuelles et les données temporelles de crête précédant les données temporelles de crête actuelles pour générer une abscisse de points de dispersion $X_i$ correspondant au second indice i, et la réalisation d'un calcul de différence absolue sur les données temporelles de crête actuelles et les données temporelles de crête actuelles suivant les données temporelles de crête actuelles pour générer une ordonnée de points de dispersion $Y_i$ correspondant au second indice i, les coordonnées bidimensionnelles de points de dispersion $XY_i$ étant composées de l'abscisse de points de dispersion Xi et de l'ordonnée de points de dispersion $Y_i$, les coordonnées bidimensionnelles de points de dispersion $XY_i$ étant $(X_i, Y_i)$, le premier indice i' étant un numéro d'indice des données temporelles de crête, et la plage de valeurs du premier indice i' étant de 2 à n-1, le second indice i étant un numéro d'indice des coordonnées bidimensionnelles de points de dispersion, $XY_i$ i=i'-1, et la plage de valeurs du second indice i étant de 1 à n-2 ; n-2 coordonnées bidimensionnelles $XY_i$ de points de dispersion formant la séquence de coordonnées bidimensionnelles de points de dispersion, la séquence de coordonnées bidimensionnelles de points de dispersion étant $(XY_i,... XY_i,... XY_{n-2})$ ; et la conservation uniquement de l'une des coordonnées bidimensionnelles $XY_i$ de points de dispersion identiques dans la séquence de coordonnées bidimensionnelles de points de dispersion, la suppression de la coordonnée bidimensionnelle $XY_i$ de points de dispersion dont l'abscisse $X_i$ de points de dispersion dépasse la valeur maximale de l'intervalle inter-battements, et la suppression de la coordonnée bidimensionnelle $XY_i$ de points de dispersion dont l'ordonnée $Y_i$ de points de dispersion dépasse la valeur maximale de l'intervalle inter-battements.

2. Procédé de classification de diagramme de dispersion pour un signal PPG selon la revendication 1, **caractérisé en ce que** la réalisation d'un traitement de confirmation de résolution de diagramme de dispersion selon la fréquence d'échantillonnage et une valeur maximale préétablie d'intervalle inter-battements pour générer une résolution de diagramme de dispersion comprend spécifiquement :

le calcul du nombre maximal de pixels en fonction de la fréquence d'échantillonnage et de la valeur maximale de l'intervalle inter-battements pour générer un nombre maximal de pixels a, a=fréquence d'échantillonnage * valeur maximale de l'intervalle inter-battements ; et le nombre de pixels horizontaux X de la résolution du diagramme de dispersion étant fixé au nombre maximal de pixels a, et le nombre de pixels verticaux Y de la résolution du diagramme de dispersion étant fixé au nombre maximal de pixels a, et la résolution du diagramme de dispersion étant = X*Y = a*a.

3. Procédé de classification de diagramme de dispersion pour un signal PPG selon la revendication 2, **caractérisé en ce que** la réalisation d'un traitement d'initialisation de diagramme de dispersion selon la résolution de diagramme de dispersion pour générer un tenseur bidimensionnel de diagramme de dispersion comprend spécifiquement :

le réglage du tenseur bidimensionnel du diagramme de dispersion selon la résolution du diagramme de dispersion, la forme du tenseur bidimensionnel du diagramme de dispersion étant $H_1 \times W_1$, $H_1$ étant un paramètre bidimensionnel du tenseur bidimensionnel du diagramme de dispersion, et $H_1=Y=a$, $W_1$ étant un paramètre unidimensionnel du tenseur bidimensionnel du diagramme de dispersion, et $W_1=X=a$, le tenseur bidimensionnel de diagramme de dispersion comprenant $H_1*W_1$ données de pixels $D_{s,z}$, et la valeur des données de pixel $D_{s,z}$ étant une première valeur de pixel prédéfinie, S étant un indice horizontal des données de pixel $D_{s,z}$, la plage de valeur de S étant de 1 à $W_1$, Z étant un indice vertical des données de pixel $D_{s,z}$, et la plage de valeurs de Z étant de 1 à $H_1$.

4. Procédé de classification de diagramme de dispersion pour un signal PPG selon la revendication 3, **caractérisé en ce que** la réalisation d'un traitement de marquage de points de dispersion sur le tenseur bidimensionnel de diagramme de dispersion selon la séquence de coordonnées bidimensionnelles de points de dispersion comprend :

dans le tenseur bidimensionnel du diagramme de dispersion, la valeur des données de pixel $D_{s,z}$ correspondant aux coordonnées bidimensionnelles de points de dispersion $XY_i$ de la séquence de coordonnées bidimensionnelles de points de dispersion est fixée à la seconde valeur prédéfinie de pixel, l'indice horizontal S des données de pixel $D_{s,z}$ étant identique à l'abscisse de points de dispersion $X_i$ des coordonnées bidimensionnelles de points de dispersion $XY_i$, l'indice vertical Z des données de pixel $D_{s,z}$ étant identique à l'ordonnée de points de dispersion $Y_i$ des coordonnées bidimensionnelles de points de dispersion $XY_i$.

5. Procédé de classification de diagramme de dispersion pour un signal PPG selon la revendication 3, **caractérisé en ce que** l'utilisation d'un réseau neuronal convolutif d'un réseau d'intelligence artificielle pour effectuer un calcul de regroupement convolutif multicouche sur le tenseur bidimensionnel de diagramme de dispersion pour générer un tenseur de sortie quadridimensionnel comprend spécifiquement :

selon un format de données d'entrée de tenseur quadridimensionnel du réseau neuronal convolutif, l'augmentation de la forme du tenseur bidimensionnel du diagramme de dispersion d'une forme de tenseur bidimensionnel à une forme de tenseur quadridimensionnel pour générer un tenseur d'entrée quadridimensionnel, la forme du tenseur d'entrée quadridimensionnel étant $B_1 \times H_2 \times W_2 \times C_1$, $B_1$ étant un paramètre quadridimensionnel du tenseur d'entrée quadridimensionnel, et $B_1=1$, $H_2$ étant un paramètre tridimensionnel du tenseur d'entrée quadridimensionnel, et $H_2=H_1$, $W_2$ étant un paramètre bidimensionnel du tenseur d'entrée quadridimensionnel, et $W_2=W_1$, $C_1$ étant un paramètre unidimensionnel du tenseur d'entrée quadridimensionnel, et $C_1=1$ ; et

l'envoi du tenseur d'entrée quadridimensionnel dans une première couche de réseau convolutif du réseau neuronal convolutif pour un calcul de regroupement convolutif de première couche afin de générer un premier tenseur quadridimensionnel, puis l'envoi du premier tenseur quadridimensionnel dans une seconde couche de réseau convolutif du réseau neuronal convolutif pour un calcul de regroupement convolutif de seconde fin de générer un second tenseur quadridimensionnel, jusqu'à, finalement, l'envoi d'un avant-dernier tenseur quadridimensionnel dans une dernière couche de réseau convolutif du réseau neuronal convolutif pour un calcul de regroupement convolutif de dernière couche afin de générer le tenseur de sortie quadridimensionnel, le réseau neuronal convolutif comprenant une pluralité de couches de réseau convolutif, la couche de réseau convolutif comprenant une couche convolutive et une couche de regroupement, la forme du tenseur de sortie quadridimensionnel étant $B_2 \times H_3 \times W_3 \times C_2$, $B_2$ étant un paramètre quadridimensionnel du tenseur de sortie quadridimensionnel, et $B_2=B_1=1$, $H_3$ étant un paramètre tridimensionnel du tenseur de sortie quadridimensionnel, $W_3$ étant un paramètre bidimensionnel du tenseur de sortie quadridimensionnel, et $C_2$ étant un paramètre unidimensionnel du tenseur de sortie quadridimensionnel,

l'envoi du tenseur d'entrée quadridimensionnel dans une première couche de réseau convolutif du réseau neuronal convolutif pour le calcul de regroupement convolutif de première couche afin de générer un premier tenseur d'entrée quadridimensionnel consistant à envoyer le tenseur d'entrée quadridimensionnel à une première couche de réseau convolutif de la première couche de réseau convolutif pour le premier calcul convolutif afin de générer un premier tenseur quadridimensionnel convolutif, puis l'envoi du premier tenseur quadridimensionnel convolutif dans une première couche de regroupement de la première couche de réseau convolutif pour un premier calcul de regroupement afin de générer le premier tenseur quadridimensionnel.

6. Procédé de classification par diagramme de dispersion pour un signal PPG selon la revendication 5, **caractérisé en ce que** l'utilisation du réseau neuronal entièrement connecté du réseau d'intelligence artificielle pour effectuer un calcul de connexion complète multicouche sur le tenseur de sortie quadridimensionnel pour générer un tenseur de sortie bidimensionnel comprend spécifiquement :

selon un format de données d'entrée de tenseur bidimensionnel du réseau neuronal entièrement connecté, la réduction de la forme du tenseur de sortie quadridimensionnel d'une forme de tenseur quadridimensionnel à une forme de tenseur bidimensionnel pour générer un tenseur d'entrée bidimensionnel, la forme du tenseur d'entrée bidimensionnel étant $B_3 x W_4$, $B_3$ étant un paramètre bidimensionnel du tenseur d'entrée bidimensionnel, et $B_3=B_2=1$, $W_4$ étant un paramètre unidimensionnel du tenseur d'entrée bidimensionnel, et $W_4=H_3*W_3*C_2$ ; et

l'envoi du tenseur d'entrée bidimensionnel dans une première couche entièrement connectée du réseau neuronal entièrement connecté pour le calcul de connexion complète de la première couche afin de générer un premier tenseur bidimensionnel, puis l'envoi du premier tenseur bidimensionnel à une seconde couche entièrement connectée du réseau neuronal entièrement connecté pour le calcul de connexion complète de la seconde couche afin de générer un second tenseur bidimensionnel, jusqu'à l'envoi final d'un avant-dernier tenseur bidimensionnel dans une dernière couche entièrement connectée du réseau neuronal entièrement connecté pour le calcul de connexion complète de la dernière couche afin de générer le tenseur de sortie bidimensionnel, le réseau neuronal entièrement connecté comprenant une pluralité de couches entièrement connectées, la forme du tenseur de sortie bidimensionnel étant $B_4 x W_5$, $B_4$ étant un paramètre bidimensionnel du tenseur de sortie bidimensionnel, et $B_4=B_3=1$, $W_5$ étant un paramètre unidimensionnel du tenseur de sortie bidimensionnel, et $W_5=2$, et le tenseur de sortie bidimensionnel comprenant deux données : les données de poids raisonnable de classification et les données de poids déraisonnable de classification.

**7.** Procédé de classification par diagramme de dispersion pour un signal PPG selon la revendication 6, **caractérisé en ce que** l'utilisation de la couche de traitement de normalisation du réseau d'intelligence artificielle pour effectuer un calcul d'indice de normalisation sur le tenseur de sortie bidimensionnel pour générer un tenseur bidimensionnel de normalisation comprend spécifiquement :

l'envoi du tenseur de sortie bidimensionnel dans la couche de traitement de normalisation pour le calcul de l'indice de normalisation des données de poids raisonnable de classification et des données de poids déraisonnable de classification la génération d'une probabilité raisonnable de classification et d'une probabilité déraisonnable de classification, la somme de la probabilité raisonnable de classification et de la probabilité déraisonnable de classification étant de 1 ; et

la formation du tenseur bidimensionnel de normalisation par la probabilité raisonnable de classification et la probabilité déraisonnable de classification, la forme du tenseur bidimensionnel de normalisation étant $B_5 x W_6$,
$B_3$ étant un paramètre bidimensionnel du tenseur bidimensionnel de normalisation, et $B_5=B_4=1$, $W_6$ étant un paramètre unidimensionnel du tenseur bidimensionnel de normalisation, et $W_6=W_5=2$, et le tenseur bidimensionnel de normalisation comprenant deux données : la probabilité raisonnable de classification et la probabilité déraisonnable de classification.

**8.** Procédé de classification par diagramme de dispersion pour un signal PPG selon la revendication 7, **caractérisé en ce que** la réalisation d'un traitement de confirmation de classification selon le tenseur bidimensionnel de normalisation pour générer des données de confirmation (les données de confirmation comprend des informations raisonnables de classification et des informations déraisonnables de classification) comprend spécifiquement :

l'adoption des informations raisonnables de classification comme données de confirmation lorsque la probabilité raisonnable de classification est supérieure à la probabilité déraisonnable de classification ; et

l'adoption des informations déraisonnables de classification comme données de confirmation lorsque la probabilité raisonnable de classification est inférieure à la probabilité déraisonnable de classification.

**9.** Dispositif de classification par diagramme de dispersion pour un signal de photopléthysmographie (PPG), **caractérisé en ce que** le dispositif comprend :

un module d'acquisition (51) pour acquérir le signal PPG ;
un module d'échantillonnage (52) pour effectuer un traitement d'échantillonnage de signal sur le signal PPG selon une fréquence d'échantillonnage préétablie afin de générer un signal d'échantillonnage PPG ;
un module de diagramme de dispersion (53) permettant d'effectuer un traitement de préparation de coordonnées bidimensionnelles de points de dispersion en fonction du signal d'échantillonnage PPG afin de générer une séquence de coordonnées bidimensionnelles de points de dispersion ; d'effectuer un traitement de confirmation de résolution de diagramme de dispersion en fonction de la fréquence d'échantillonnage et d'une valeur maximale prédéfinie d'un intervalle inter-battements afin de générer une résolution de diagramme de dispersion ; d'effectuer un traitement d'initialisation de diagramme de dispersion en fonction de la résolution du diagramme de dispersion pour générer un tenseur bidimensionnel de diagramme de dispersion ;

et d'effectuer un traitement de marquage des points de dispersion sur le tenseur bidimensionnel du diagramme de dispersion selon la séquence de coordonnées bidimensionnelles de points de dispersion ;

un module de calcul d'intelligence artificielle (54) permettant d'utiliser un réseau neuronal convolutif d'un réseau d'intelligence artificielle pour effectuer un calcul de regroupement convolutif multicouche sur le tenseur bidimensionnel de diagramme de dispersion afin de générer un tenseur de sortie quadridimensionnel ; d'utiliser un réseau neuronal entièrement connecté du réseau d'intelligence artificielle pour effectuer un calcul de connexion complète multicouche sur le tenseur de sortie quadridimensionnel afin de générer un tenseur de sortie bidimensionnel ; et d'utiliser une couche de traitement de normalisation du réseau d'intelligence artificielle pour effectuer un calcul d'indice de normalisation sur le tenseur de sortie bidimensionnel afin de générer un tenseur de sortie bidimensionnel, le réseau d'intelligence artificielle comprenant le réseau neuronal convolutif, le réseau neuronal entièrement connecté et la couche de traitement de normalisation ; et

un module de confirmation de classification (55) permet d'effectuer un traitement de confirmation de classification selon le tenseur bidimensionnel de normalisation afin de générer des données de confirmation, les données de confirmation comprenant des informations raisonnables de classification et des informations déraisonnables de classification.

10. Produit de programme informatique comprenant un code de programme informatique qui, lorsqu'il est exécuté par un ordinateur, amène l'ordinateur à exécuter le procédé selon l'une quelconque des revendications 1 à 8.

11. Support de stockage lisible par ordinateur, qui stocke des instructions informatiques qui, lorsqu'elles sont exécutées par un ordinateur, amènent l'ordinateur à exécuter le procédé selon l'une quelconque des revendications 1 à 8.

| | |
|---|---|
| Acquiring the PPG signal | 1 |

$\downarrow$

| | |
|---|---|
| Performing signal sampling processing on the PPG signal according to a preset sampling frequency to generate a PPG sampling signal | 2 |

$\downarrow$

| | |
|---|---|
| Performing scatter point two-dimensional coordinate preparation processing according to the PPG sampling signal to generate a scatter point two-dimensional coordinate sequence | 3 |

$\downarrow$

| | |
|---|---|
| Performing scatter diagram resolution confirmation processing according to the sampling frequency and a preset maximum value of an inter-beat interval to generate a scatter diagram resolution | 4 |

$\downarrow$

| | |
|---|---|
| Performing scatter diagram initialization processing according to the scatter diagram resolution to generate a scatter diagram two-dimensional tensor | 5 |

$\downarrow$

| | |
|---|---|
| Performing scatter point marking processing on the scatter diagram two-dimensional tensor according to the scatter point two-dimensional coordinate sequence | 6 |

$\downarrow$

| | |
|---|---|
| Using a convolutional neural network of an artificial intelligence network to perform multilayer convolution pooling calculation on the scatter diagram two-dimensional tensor to generate a four-dimensional output tensor | 7 |

$\downarrow$

| | |
|---|---|
| Using the fully connected neural network of the artificial intelligence network to perform multilayer full connection calculation on the four-dimensional output tensor to generate a two-dimensional output tensor | 8 |

$\downarrow$

| | |
|---|---|
| Using the normalization processing layer of the artificial intelligence network to perform normalization index calculation on the two-dimensional output tensor to generate a normalization two-dimensional tensor | 9 |

$\downarrow$

| | |
|---|---|
| Performing classification confirmation according to the normalization two-dimensional tensor to generate confirmation data, the confirmation data comprising classification reasonable information and classification unreasonable information | 10 |

Fig. 1

Fig. 2

Fig. 3

Fig. 4a

Fig. 4b

Fig. 4c

Acquisition module    51

Sampling module    52

Scatter diagram module    53

Artificial intelligence calculation module    54

Classification confirmation module    55

Fig. 5

| Processor | ⌇ 61 | Power supply | ⌇ 64 |

65 ⌇

Memory ⌇ 62

| Transceiver | ⌇ 63 | Communication port | ⌇ 66 |

Fig. 6

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 202011086771
- WO 2019191487 A1
- WO 2020125078 A1
- CN 112070076 A
- CN 111291727 A
- CN 108784680 A
- CN 111248880 A